(19) Europäisches Patentamt
European Patent Office
Office européen des brevets

(11) EP 1 995 680 A2

(12) **EUROPEAN PATENT APPLICATION**

(43) Date of publication:
**26.11.2008 Bulletin 2008/48**

(51) Int Cl.:
***G06F 19/00*** *(2006.01)*

(21) Application number: **08009364.4**

(22) Date of filing: **21.05.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **23.05.2007 JP 2007136412**

(71) Applicant: **SYSMEX CORPORATION**
**Kobe-shi,**
**Hyogo 651-0073 (JP)**

(72) Inventors:
- **Kouchi, Yasuhiro**
**Chuo-ku**
**Kobe-shi**
**Hyogo 651-0073 (JP)**
- **Saitou, Takeo**
**Chuo-ku**
**Kobe-shi**
**Hyogo 651-0073 (JP)**
- **Seike, Masayoshi**
**Chuo-ku**
**Kobe-shi**
**Hyogo 651-0073 (JP)**

(74) Representative: **HOFFMANN EITLE**
**Patent- und Rechtsanwälte**
**Arabellastrasse 4**
**81925 München (DE)**

(54) **Medical diagnosis support computer system, computer program, and server computer**

(57) A medical diagnosis support computer system for supporting diagnosis of a patient by a doctor, the system comprising: a display device; and a controller in communication with the display device, the controller comprising a processor configured to build a biological model representing biological functions of the patient on a computer, generate pathological condition information of the patient based on the biological model, display a doctor screen and a patient screen in a switchable manner on the display device; wherein the doctor screen is configured to simultaneously display a screen region for displaying the pathological condition information of the patient and a screen region for displaying one or more related information related to the patient; and the patient screen is configured to selectively display a display content between the pathological condition information of the patient and one or more related information related to the patient is disclosed.

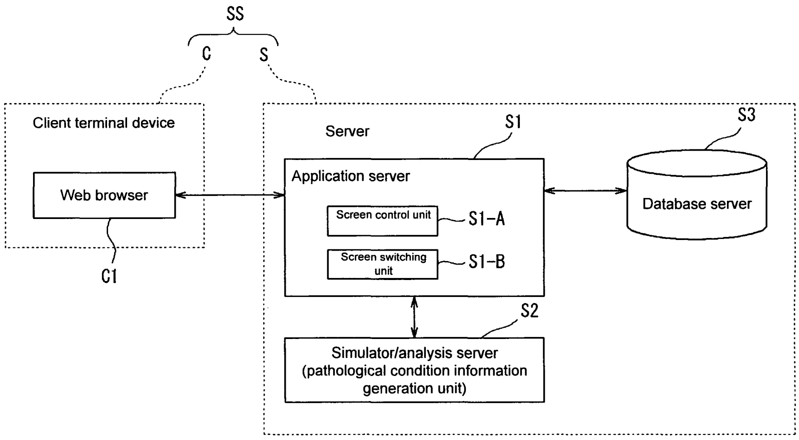


**FIG. 1**

EP 1 995 680 A2

## Description

FIELD OF THE INVENTION

The present invention relates to a medical diagnosis support computer system used to support diagnosis of diabetes or the like, a computer program, and a server computer.

BACKGROUND OF THE INVENTION

In treating diseases, various tests are usually performed on patients in addition to inquiry by doctors.
The doctors are currently selecting the treatment methods relying on his/her experience and intuition based on information for making a decision such as test results and clinical findings of the patient.
Therefore, it is presumed that medical examinations by doctors will be more accurately carried out if information useful for medical examination is provided by a computer.
A system for supporting medical examination includes a system for predicting blood glucose level as disclosed in Japanese Laid-Open Patent Publication No. 10-332704 and Japanese Laid-Open Patent Publication No. 11-296598.
Such systems support the medical examination by predicting change in the blood glucose level of a patient and providing the predicted blood glucose level to the doctor.
When choosing an appropriate treatment method, it is desired that the doctor appropriately understands the factors constituting the cause of various symptoms of the diseases. If the factors are appropriately understood, a more appropriate treatment can be expected by carrying out treatments to improve such factor.
However, the data that the doctor can use to understand the factor is only the test values obtained by giving tests to the patient.
The disease is hoped such that the doctor can understand the factor with only the test values, but in some diseases, it is significantly difficult to appropriately understand the factor with only the test values.
For example, in the case of diabetes, "blood glucose level" is used as an index for indicating the extent of the disease. However, the "blood glucose level" is merely a result, and it is not easy to accurately understand the pathological conditions such as insulin deficiency, peripheral insulin resistance, lowering in uptake of hepatic glucose, and increase in release of hepatic glucose from the clinical findings and the like.
Therefore, it is still difficult to accurately determine the pathological conditions of the patient if the predicted value of the blood glucose level is merely provided to the doctor as described in Japanese Laid-Open Patent Publication No. 10-332704 and Japanese Laid-Open Patent Publication No. 11-296598.
In diabetes, heart disease and the like, the specialist determines the pathological condition of the patient by using oral glucose tolerance test, electrocardiogram, and test results such as blood pressure, pulse measurement, blood test, and the like, but abundant experience is required in order to accurately determine the pathological condition from the test results, and this accurate determination is difficult for non-specialists.
There is demanded a system having an effect of teaching inexperienced doctors and non-specialists so as to be able accurately determine the pathological conditions from the test results.
Although there is demanded a support system for the doctor to accurately determine the pathological condition of the patient, such system is suitable if it can also be used to provide necessary information to the patients.
In other words, it is important for the patients to understand his/her condition in terms of enhancing the treatment effect, and the information provided from the system by screen display etc. are desirably viewable by doctors as well as patients.
However, if information is provided to the patient in the same manner as the manner that the information is provided to the doctor to make accurate determination on the pathological condition, the information may be too complex for the patient, thereby impairing easy understanding of the patient.
If information is provided to the patient so as to be understandable, the amount of information to be screen displayed etc. becomes small, and it makes difficult for the doctors to accurately determine the pathological condition of the patient thereby impairing easy diagnosis of the doctor.

SUMMARY OF THE INVENTION

The scope of the present invention is defined solely by the appended claims, and is not affected to any degree by the statements within this summary.
A first aspect of the present invention is a medical diagnosis support computer system for supporting diagnosis of a patient by a doctor, the system comprising: a display device; and a controller in communication with the display device, the controller comprising a processor configured to build a biological model representing biological functions of the patient on a computer, generate pathological condition information of the patient based on the biological model, display a doctor screen for the doctor and a patient screen for the patient in a switchable manner on the display device, and accept an input for switching the display between the doctor screen and the patient screen; wherein the doctor screen is configured to simultaneously display a screen region for displaying the pathological condition information of the patient and a screen region for displaying one or more related information related to the patient; and the patient screen is configured to selectively display a display content between the pathological condition information of the patient and one or more related information related to the patient.

A second aspect of the present invention is a computer program product, comprising: a computer readable medium; and instructions, on the computer readable medium, adapted to enable a general purpose computer to perform operations, comprising: building a biological model representing biological functions of the patient on a computer; generating pathological condition information of the patient based on the biological model; displaying a doctor screen for the doctor and a patient screen for the patient in a switchable manner on the display device; and receiving an input for switching the display between the doctor screen and the patient screen; wherein the doctor screen is configured to simultaneously display a screen region for displaying the pathological condition information of the patient and a screen region for displaying one or more related information related to the patient; and the patient screen is configured to selectively display a display content between the pathological condition information of the patient and one or more related information related to the patient.

A third aspect of the present invention is A medical diagnosis support computer system for supporting diagnosis of a patient by a doctor, the system comprising: a display device; and a controller in communication with the display device, the controller comprising a processor configured to build a biological model representing biological functions of the patient on a computer, generate pathological condition information of the patient based on the biological model, display a doctor screen for the doctor and a patient screen for the patient in a switchable manner on the display device, and receive an input for switching the display between the doctor screen and the patient screen; wherein the doctor screen is configured to simultaneously display a screen region for displaying the pathological condition information of the patient and a screen region for displaying one or more related information related to the patient; and the patient screen comprises a test result screen for displaying result information of a medical test of the patient without display of the pathological condition information.

A fourth aspect of the present invention is a computer program product, comprising: a computer readable medium; and instructions, on the computer readable medium, adapted to enable a general purpose computer to perform operations, comprising: building a biological model representing biological functions of the patient on a computer; generating pathological condition information of the patient based on the biological model; displaying a doctor screen for the doctor and a patient screen for the patient in a switchable manner on the display device; and receiving an input for switching the display between the doctor screen and the patient screen; wherein the doctor screen is configured to simultaneously display a screen region for displaying the pathological condition information of the patient and a screen region for displaying one or more related information related to the patient; and the patient screen comprises a test result screen for displaying result information of a medical test of the patient without display of the pathological condition information.

A fifth aspect of the present invention is a server computer for displaying a screen for supporting a diagnosis of a patient by a doctor on a display device of a terminal device connected by way of a network, the server computer comprising: a controller in communication with the terminal device, the controller comprising a processor configured to display a doctor screen for the doctor and a patient screen for the patient in a switchable manner on the display device of the terminal device, and switch the display between the doctor screen and the patient screen when an input for switching between the doctor screen and the patient screen is made at the terminal device; wherein the doctor screen is configured to simultaneously display a screen region for displaying pathological condition information of the patient generated based on a biological model built on a computer to represent biological functions of the patient and a screen region for displaying one or more related information related to the patient; and the patient screen is configured to selectively display a display content between the pathological condition information of the patient and one or more related information related to the patient.

A sixth aspect of the present invention is a server computer for displaying a screen for supporting a diagnosis of a patient by a doctor on a display device of a terminal device connected by way of a network, the server computer comprising: a controller in communication with the terminal device, the controller comprising a processor configured to display a doctor screen for the doctor and a patient screen for the patient in a switchable manner on the display device of the terminal device, and switch the display between the doctor screen and the patient screen when an input for switching between the doctor screen and the patient screen is made at the terminal device; wherein the doctor screen is configured to simultaneously display a screen region for displaying pathological condition information of the patient generated based on a biological model built on a computer to represent biological functions of the patient and a screen region for displaying one or more related information related to the patient; and the patient screen includes a test result screen for displaying result information of a medical test of the patient without display of the pathological condition information.

BRIEF DESCRIPTION OF THE DRAWINGS

Fig. 1 is a system configuration view of a medical diagnosis support computer system;
Fig. 2 is a block diagram showing a hardware configuration of a server;
Fig. 3 is a block diagram showing an overall configuration of a biological model;
Fig. 4 is a block diagram showing a configuration of a pancreas model of the biological model;
Fig. 5 is a block diagram showing a configuration of a hepatic model of the biological model;
Fig. 6 is a block diagram showing a configuration of an insulin kinetics model of the biological model;
Fig. 7 is a block diagram showing a configuration of a peripheral tissue model of the biological model;
Fig. 8 is a flowchart showing a parameter set generating process procedure;
Fig. 9 shows actual measured OGTT time-series data, wherein (a) is blood glucose level, and (b) is blood insulin concentration;
Fig. 10 is a configuration of a template database DB1;
Fig. 11 is a template data, wherein (a) is blood glucose level and (b) is insulin concentration;
Fig. 12 is a view showing error summation of the OGTT time-series data with respect to the template T1, wherein (a) is blood glucose level and (b) is insulin concentration;
Fig. 13 is a basic screen of the system;
Fig. 14 is a screen transition view of the system;
Fig. 15 is a doctor screen;
Fig. 16 is a patient screen;
Fig. 17 is a view showing a data structure of a database;
Fig. 18 is a view showing an operation menu of the basic screen;
Fig. 19 is a patient information edit screen;
Fig. 20 is a basic test data input screen;
Fig. 21 is an OGTT data input screen;
Fig. 22 is a target value input screen;
Fig. 23 is a doctor screen;
Fig. 24 is a patient screen in which the basic test data screen region is selected;
Fig. 25 is a patient screen in which the OGTT data screen region is selected;
Fig. 26 is a patient screen in which the prescription/finding screen region is selected; and
Fig. 27 is a patient screen in which the past prescription list screen region is selected.

DETAILED DESCRIPTION OF THE PREFERRED EMBODIMENTS

The preferred embodiments of the present invention will be described hereinafter with reference to the drawings.
Embodiments of a medical diagnosis support computer system (hereinafter also simply referred to as "system") according to the present invention will now be described in detail with reference to the accompanied drawings.

[Overall configuration of system]

Fig. 1 shows a system configuration view of the case when a medical diagnosis support computer system SS is configured as a server client system. In the present embodiment, the system SS is built as a system for supporting diagnosis of diabetes.
The system SS is configured as a network system including a server computer (hereinafter also simply referred to as "server") S, and a client terminal device (hereinafter also simply referred to as "client") connected to the server S by way of Internet or network such as LAN.
The system SS may be configured by one computer.
The client C is used by a user such as doctor, laboratory technician, and the like. The client C is configured by a computer including a display device (display) and an input device (keyboard or mouse etc.). A printer (not shown) is connected to the client C, so that display screen etc. of the client can be printed.
The client C includes a Web browser C1. The Web browser C1 serves as a user interface of the system SS. That is, the screen generated in the server S and transmitted to the client C is output on the Web browser C1. The user can input and perform necessary operations on the Web browser C1.
The server S has a function serving as an application server S1, a function serving as a simulator/analysis server S2, and a function serving as a database server S3. Each function S1 to S3 may be realized with one computer, or may be realized by a plurality of computers connected network.

In the present embodiment, the application server S1 has a central function to have the network system SS function as the medical diagnosis support system, and has a function of generating a screen to be displayed on the Web browser C1 and transmitting the same to the client terminal C, and of accepting the information input in the Web browser C1 from the client terminal C.

Thus, a computer program (application program) for realizing a function of generating a user interface screen to be displayed on the Web browser C1, a function of accepting information from the client terminal C, and a function of performing other necessary processes is loaded on the application server S1 so as to be executed by a computer.

The client terminal C may download a program such as java (registered trademark) applet for realizing some or all of the functions of the screen displayed on the Web browser C1 from the server S, and display the screen on the Web browser C1.

The simulator/analysis server (pathological condition information analysis server) S2 builds a biological model representing the biological functions of a patient on a computer (server S), and generates pathological condition information (pathological condition information of diabetes) of the patient based on the biological model.

In the present embodiment, the biological model is built as a mathematical model on the computer including a plurality of parameters related to the biological functions to represent the entire human body or biological functions of a certain disease. The simulator/analysis server S2 performs simulation on the disease (diabetes) of a specific patient based on the biological model, and acquires the pathological condition information of the relevant patient by simulation.

A pathological condition simulator/analysis program (pathological information analysis computer program) is loaded on the simulator/analysis server (pathological condition information generation server; pathological condition information generation unit) S2 to be executed by a computer, and the simulation and the analysis function are realized by the relevant program.

A database having various data DB1 to DB7 of test result etc. of the patient is built in the database server S3.

Fig. 2 is a block diagram showing hardware configuration of the server S. The server S is composed of a computer mainly including a main body S110, a display S120, and an input device S130. The main body S110 is mainly configured by a CPU 110a, a ROM S110b, a RAM S110c, a hard disk S110d, a readout device S110e, an input/output interface S110f, and an image output interface S110h, wherein the CPU S110a, the ROM S110b, the RAM S110c, the hard disk S110d, the readout device S110e, the input/output interface S110f, and the image output interface S110h are data-communicably connected by a bus S110i.

The CPU S110a is capable of executing a computer program recorded in the ROM S110b and a computer program loaded in the RAM S110c.

The CPU S110a executes an application program 140a for realizing the application server S1 function, a program for realizing the simulator/analysis server S2 function, and a program for realizing the database server function, to realize each function block as described below, whereby the computer functions as the server S of the present embodiment.

The ROM S110b includes mask ROM, PROM, EPROM, EEPROM, etc. and is recoded with computer programs executed by the CPU 110a and data used for the programs.

The RAM S110c comprises SRAM, DRAM, etc. The RAM S110c is used to read out computer programs recorded in the ROM S110b and the hard disk S110d. The RAM 110c is used as a work area of the CPU S110a when these computer programs are executed.

The hard disk S110d is installed with an operating system and an application program, etc., as well as various computer programs to be executed by the CPU 110a, and data used for executing the computer programs.

The readout device S110e which comprises a flexible disk drive, a CD-ROM drive or DVD-ROM drive is capable of reading out a computer program or data recorded in a portable recording media S140. The portable recording media S140 stores the computer program for the computer to function as the system of the present invention, wherein the computer reads out the necessary application program S140a etc. from the portable recording media S140 and installs the relevant application program S140a etc. in the hard disk S110d.

The computer program such as the application program S140a is not only provided by the portable recording media S140, and may be provided through an electric communication line (wired or wireless) from external devices which are communicably connected to the computer via the electric communication line. For example, the application program S140a etc. may be stored in a hard disk in an application program providing server computer on the Internet, so that the computer program can be downloaded by accessing the server computer and installed in the hard disc S110d.

The hard disk 110d is installed with an operating system which provides a graphical user interface environment, e.g. Windows (registered trademark) manufactured by US Microsoft Corp. In the following description, the computer program described in the present embodiment is assumed as operating on the operating system.

The input/output interface S110f includes a serial interface such as USB, IEEE1394, and RS-232C; a parallel interface such as SCSI, IDE, and IEEE1284; and an analog interface such as D/A converter and A/D converter. The input/output interface S110f is connected to the input device S130 including a keyboard and a mouse, and users can use the input device S130 to input data into the computer.

The image output interface S110h is connected to the display S120 configured by LCD, CRT or the like so that picture signals corresponding to image data provided from the CPU S110a are output to the display S120. The display S120 displays an image (screen) based on the input picture signals.
The hardware configuration of the client terminal C is the same as the hardware configuration of the server S.

[Simulator/analysis server S2]

The simulator/analysis server (pathological condition information generation server; pathological condition information generation unit) S2 will now be described.

[Biological model]

Fig. 3 is a block diagram showing an overall configuration of one example of a biological model used in the pathological condition simulation according to the present embodiment. The biological model particularly simulates biological organs associated with diabetes, and comprises a pancreas block 1, a hepatic block 2, an insulin kinetics block 3, and a peripheral tissue block 4.
Each block 1, 2, 3, 4 has input and output. That is, as to the pancreas block 1, a blood glucose level 6 is set as input and an insulin secretion rate 7 is set as output.
As to the hepatic block 2, glucose absorption 5 from digestive tract, the blood glucose level 6 and then insulin secretion rate 7 are set as input, and net glucose release 8 and post liver insulin 9 are set as output.
As to the insulin kinetics block 3, post liver insulin 9 is set as input and insulin concentration 10 at the peripheral tissues is set as output.
As to the peripheral tissue block 4, the net glucose release 8, and the insulin concentration 10 at the peripheral tissues are set as input, and the blood glucose level 6 is set as output.
Glucose absorption 5 is data provided from outside of the biological model (database server S3). In the present embodiment, data related to glucose absorption is registered in the database S3 as actual test data

(biological response).

Furthermore, the function blocks 1 to 4 are respectively realized when the computer program is executed by the CPU of the server S2.
Next, each of above-mentioned blocks will be described in detail. FGB and Ws indicate a fasting blood glucose level (FGB = BG (0)) and the patient's weight, respectively, and DVg and DVi indicate a distribution capacity volume with respect to glucose and a distribution capacity volume with respect to insulin, respectively.

[Pancreas block of biological model]

Relationship between input and output of the pancreas block 1 may be expressed using the following differential equation (1). The relationship can also be represented using a block diagram as in Fig. 6 equivalent to the differential equation (1).

Differential equation (1):

$$dY/dt = -\alpha\{Y(t) - \beta(BG(t) - h)\} \quad (BG(t) > h)$$

$$= -\alpha Y(t) \quad (BG(t) <= h)$$

$$dX/dt = -M \bullet X(t) + Y(t)$$

$$SR(t) = M \bullet X(t)$$

Variables:

BG(t): blood glucose level
X(t): total amount of insulin capable of being secreted from pancreas
Y(t): supply rate of insulin newly supplied to X(t) with respect to glucose stimulation

SR(t): insulin secretion rate from pancreas Parameters:
h: threshold of glucose concentration capable of stimulating insulin supply
$\alpha$: following performance to glucose stimulation
$\beta$: sensitivity to glucose stimulation
M: secretion rate per unit concentration

The blood glucose level 6 which is the input to the pancreas block 1 in Fig. 3 corresponds to BG(t), and the insulin secretion rate 7 which is the output corresponds to SR(t).

In the block diagram in Fig. 6, numeral 6 indicates blood glucose level BG (t); 7 indicates insulin secretion rate SR (t) from pancreas; 12 indicates glucose concentration threshold h capable of stimulating insulin supply; 13 indicates sensitivity to glucose stimulation $\beta$; 14 indicates following performance to glucose stimulation $\alpha$; 15 indicates integral element; 16 indicates supply rate Y(t) of newly supplied insulin with respect to glucose stimulation; 17 indicates integral element; 18 indicates total amount of insulin X(t) capable of being secreted from pancreas; and 19 indicates secretion rate M per unit concentration.

[Hepatic block of biological model]

Relationship between input and output of the hepatic block 2 may be described using the following differential equation (2). The relationship can also be represented using a block diagram as in Fig. 7 equivalent to the differential equation (2).

Differential equation (2):

$$dI_4(t)/dt = \alpha 2\{-A_3 I_4(t) + (1-A_7)\bullet SR(t)\}$$

$$Goff(FGB) = f1 \quad (FGB<f3)$$

$$= f1 + f2\bullet(FGB-f3)$$

$$(FGB>=f3)$$

$$Func1(FGB) = f4 - f5\bullet(FGB-f6)$$

$$Func2(FGB)=f7/FGB$$

$$b1(I_4(t))= f8\{1 + f9\bullet I4(t)\}$$

$$HGU(t)=r\bullet Func1(FGB)\bullet b1(I_4(t))\bullet RG(t)+ (1-r)\bullet Kh\bullet BG(t)\bullet I_4(t) \quad (HGU(t)>=0)$$

$$HGP(t) = I_{4off}\bullet Func2(FGB)\bullet b2+G_{off}(FGB)-I_4(t)\bullet Func2(FGB)\bullet b2 \quad (HGP(t)>= 0)$$

$$SGO(t) =RG(t)+HGP(t)-HGU(t)$$

$$SRpost(t) = A_7 SR(t)$$

Variables:

BG(t): blood glucose level (glucose concentration per unit volume of blood)
SR(t): insulin secretion rate from pancreas

SRpost(t): post hepatic insulin
RG(t): glucose absorption from digestive tract
HGP(t): hapatic glucose release
HGU (t): hepatic glucose uptake
SGO (t): net glucose from liver
$I_4$(t): hepatic insulin concentration

Parameter:

Kh: insulin dependent glucose uptake rate in liver per unit glucose
$A_7$: insulin uptake rate in liver
Goff: glucose release rate to basal metabolism
b2: adjustment term for hepatic glucose release suppression rate
r: insulin independent hepatic glucose uptake distribution rate
$\alpha$2: following performance with respect to insulin stimulation
$I_{4off}$: insulin concentration threshold at which hepatic glucose release is suppressed

Function:

Goff (FBG): glucose release rate to basal metabolism
Func1 (FBG): hepatic glucose uptake rate to glucose stimulation from digestive tract
Func2 (FBG): hepatic glucose release suppression rate to insulin stimulation
f1 to f9: constants used to express the above-mentioned three elements
b1($I_4$(t)): adjustment item for hepatic glucose uptake rate

Here, the glucose absorption 5 from digestive tract which is input to the hepatic block in Fig. 3 corresponds to RG(t), the blood glucose level 6 corresponds to BG(t) and the insulin secretion rate 7 corresponds to SR(t), and furthermore, the net glucose release 8 which is the output corresponds to SGO(t) and the post liver insulin 9 corresponds to SRpost(t).

In a block diagram in Fig. 7, numeral 5 indicates glucose absorption RG(t) from digestive tract; 6 indicates blood glucose level BG(t); 7 indicates insulin secretion rate SR(t) from pancreas; 8 indicates net glucose SGO(t) from liver; 9 indicates post liver insulin SRpost(t); 24 indicates insulin passage rate (1-$A_7$) of liver; 25 indicates following performance to insulin stimulation $\alpha$2; 26 indicates post liver insulin distribution rate $A_3$; 27 indicates integral element; 28 indicates hepatic insulin concentration $I_4$(t); 9 indicates insulin-dependant hepatic glucose uptake distribution rate (1-r); 30 indicates insulin-dependent glucose uptake rate Kh in liver per unit glucose; 31 indicates insulin-independent hepatic glucose uptake distribution rate r; 32 indicates hepatic glucose uptake rate to glucose stimulation from digestive tract Func1(FGB); 33 indicates adjustment item for hepatic uptake rate b1 ($I_4$(t)); 34 indicates hepatic glucose uptake HGU(t); 35 indicates insulin concentration threshold at which hepatic glucose release is suppressed $I_{4off}$; 36 indicates hepatic release suppression rate (FGB) to insulin stimulation Func2; 37 indicates adjustment items hepatic glucose release suppression rate b2; 38 indicates glucose release rate HGP(t) to basal metabolism; 39 indicates hepatic glucose release HGP(t), and 40 indicates insulin uptake rate $A_7$ in liver.

[Insulin kinetics block of biological model]

Relationship between input and output of the insulin kinetics secretion may be described using the following differential equation (3). The relationship can also be represented using a block diagram as in Fig. 8 equivalent to the differential equation (3).

Differential equation (3)

$$dI_1(t)/dt = -A_3I_1(t)+A_5I_2(t)+A_4I_3(t)+SRpost(t)$$

$$dI_2(t)/dt= A_6I_1(t)- A_5I_2(t)$$

$$dI_3(t)/dt=A_2I_1(t) - A_1I_3(t)$$

Variables:

SRpost(t): post hepatic insulin
$I_1(t)$: blood insulin concentration
$I_2(t)$: insulin concentration in insulin independent tissues
$I_3(t)$: insulin concentration in peripheral tissues Parameters:

$A_1$: insulin disappearance rate in peripheral tissues
$A_2$: insulin distribution rate to peripheral tissues
$A_3$: post hepatic insulin distribution rate
$A_4$: post peripheral tissue insulin flow out rate
$A_5$: insulin disappearance rate in insulin independent tissues
$A_6$: insulin distribution rate to insulin independent tissues

Here, the post liver insulin 9 which is the input to the insulin kinetics block in Fig. 3 corresponds to SRpost(t), and the insulin concentration 10, which is the output, at the peripheral tissue corresponds to $I_3(t)$.

In a block diagram in Fig. 8, numeral 9 indicates post liver insulin SRpost (t); 10 indicates insulin concentration $I_3(t)$ in peripheral tissue; 50 indicates integral element; 51 indicates post liver insulin distribution rate $A_3$; 52 indicates blood insulin concentration $I_1(t)$; 53 indicates insulin distribution rate $A_2$ to peripheral tissues; 54 indicates integral element; 55 indicates insulin disappearance rate $A_1$ in peripheral tissue; 56 indicates post peripheral tissue insulin flow out rate $A_4$; 57 indicates insulin distribution rate $A_6$ to insulin independent tissue; 58 indicates integral element; 59 indicates insulin concentration in insulin independent tissue $I_2(t)$; 60 indicates insulin disappearance rate $A_5$ in insulin independent tissue.

[Peripheral tissue block of biological model]

Relationship between input and output of the peripheral tissue block 4 may be described using the following differential equation (4). The relationship can also be represented using a block diagram as in Fig. 9 equivalent to the differential equation (4).

Differential equation (4)

$$dBG'/dt=SGO(t)-u* Goff(FGB)-Kb(BG'(t)-FBG')-Kp \bullet I_3(t) \bullet BG'(t)$$

Variables:

BG'(t): blood glucose level (glucose concentration per unit weight)
(BG[mg/d1], BG'[mg/kg])
SGO(t): net glucose from liver
$I_3(t)$: insulin concentration in peripheral tissues
FBG': fasting blood glucose (provided that FBG'=BG(0))

Parameters:

Kb: insulin independent glucose consumption rate in peripheral tissues
Kp: insulin dependent glucose consumption rate in peripheral tissues per unit insulin and per unit glucose
u: ratio of insulin independent glucose consumption to basal metabolism in glucose release rate to basal metabolism

Functions:

Goff(FGB): glucose release rate to basal metabolism f1 to f3: constant used to express Goff

Here, the insulin concentration 10, which is the input to the peripheral tissue block in Fig. 3, in peripheral tissue

corresponds to $I_3$(t), the net glucose 8 from liver corresponds to SGO(t), and the blood glucose level 6 which is the output corresponds to BG(t).

In a block diagram in Fig. 9, numeral 6 indicates blood glucose level BG(t); 8 indicates net glucose SGO(t) from liver; 10 indicates insulin concentration $I_3$(t) in peripheral tissues; 70 indicates insulin independent glucose consumption rate to basal metabolism u*Goff(FGB); 71 indicates integral element; 72 indicates insulin independent glucose consumption rate Kb in peripheral tissues; 73 indicates insulin dependent glucose consumption rate Kp in peripheral tissues per unit insulin and per unit glucose; and 74 indicates unit conversion constant Ws/DVg.

As shown in Fig. 3, since inputs and outputs are mutually connected between the blocks constituting the present system, it is possible to calculate and simulate time-series change of blood glucose level and insulin concentration based on the mathematical formula by providing glucose absorption 5 from digestive tract.

With regard to calculation of the differential equations of the present system, e.g. E-Cell (software disclosed by Keio University) and MatLab (manufactured by The MathWorks, Inc.) may be employed, or other calculation systems may be employed.

[Biological model generating section]

To simulate the biological organs of individual patient by using the above-mentioned biological models as shown in Figs. 3 to 9, it is required to generate a biological model having characteristics suited for individual patient. More specifically, it is required to determine parameters and initial values of variables of the biological model according to the individual patient, and apply the determined parameters and initial values to the biological model, thereby generating a biological model suited for the individual patient. (Unless otherwise specified, an initial value of variable is also included in parameters to be generated.)

The server 2 (simulator/analysis server S2) of the present system SS thus has a function of obtaining an internal parameter set or a set of internal parameters of the biological model (hereinafter simply referred to also as "parameter set"), and generating the biological model applied with the obtained parameter set to realize the function as the biological model generating section. This function is also realized through the pathological condition simulator/analysis program.

The parameter set generated by the biological model generating section is applied to the biological model, and a biological model calculating section simulates the function of the biological organs and outputs the pseudo-response simulating the actual biological response (test result).

[Parameter set generating section]

In the following, description will be made for a parameter set generating section for generating the parameter set for forming a biological model that simulates the biological organ of the patient based on the actual test result (biological response) of the patient (biological body).

[OGTT time-series data input: Step S1-1]

Fig. 4 is a flowchart showing procedures in which the parameter set generating section of the system SS obtains a parameter set of the biological model. In order to obtain parameters, first, an input step of OGTT (oral glucose tolerance test) time-series data serving as an actual test result (biological response) is executed as shown in the figure (Step S1-1).

The OGTT time-series data are a result of OGTT (given amount of glucose solution is orally loaded to measure the temporal change of blood glucose level and blood insulin concentration) or the test actually performed on the patient to be simulated by a biological model, wherein the present system accepts the input as the actual biological response (actual test value) from the client terminal 3. Here, two data of OGTT glucose data (blood glucose change data) and OGTT insulin (blood insulin concentration change data) are input as OGTT time-series data.

The input OGTT time-series data are registered as "test data" in the OGTT data table DB4 (see Fig. 17(c)) of the databases of the database server S3.

Fig. 5 shows an example of the blood glucose level change data (Fig. 5(a)) and the blood insulin concentration change data (Fig. 5(b)) as the OGTT time-series data to be input.

The blood glucose level change data of Fig. 5(a) is measured data corresponding to temporal change of the blood glucose level BG (t), or one of output items in the biological model shown in Figs. 3 to 9.

The blood insulin concentration change data of Fig. 5(b) is measured data corresponding to temporal change of blood insulin concentration $I_1$(t), or one of output items in the biological model shown in Figs. 3 to 9.

[Template matching: Step S1-2]

Next, the present system SS matches the input OGTT time-series data to the template of template database DB1. The template database DB1 is one database contained in the databases 24 of the database server S3.

As shown in Fig. 10, the template database DB1 is stored in advance with a plurality sets of data in which biological model reference output values T1, T2, ... that become template is corresponded to parameter sets PS#01, PS#02 ... for generating the reference output value. To form a pair including the reference output value and the parameter set, an appropriate parameter set may be assigned to an arbitrary reference output value, or on the contrary, the output of the biological model in the case when an arbitrary parameter set is selected may be obtained by the biological simulation system.

Fig. 11 shows an example of a template (reference output value) T1. Fig. 11(a) is the blood glucose level change data serving as a template, which is reference time-series data corresponding to temporal change of the blood glucose level BG(t) or one of the output items in the biological model shown in Figs. 3 to 9. Fig. 11(b) is the blood insulin concentration change data serving as a template, which is reference time-series data corresponding to temporal change of the blood insulin concentration $I_1(t)$ or one of the output items in the biological model shown in Figs. 3 to 9.

The system SS computes similarity between each reference time-series data of the above-mentioned template database DB and OGTT time-series data. The similarity is obtained by obtaining error summation. The error summation is obtained by the following formula.

$$\begin{aligned}\text{Error summation} = {} & \alpha\Sigma|BG(0)-BGt(0)|+\beta\Sigma|PI(0)-PIt(0)| \\ & +\alpha\Sigma|BG(1)-BGt(1)|+\beta\Sigma|PI(1)-PIt(1)| \\ & +\alpha\Sigma|BG(2)-BGt(2)|+\beta\Sigma|PI(2)-PIt(2)| \\ & +\cdots \\ = {} & \alpha\{\Sigma|BG(t)-BGt(t)|\}+\beta\{\Sigma|PI(t)-PIt(t)|\}\end{aligned}$$

wherein

BG: input data blood glucose level [mg/dl]
PI: input data blood insulin concentration [μU/ml]
BGt: template blood glucose level[mg/dl]
PIt: template blood insulin concentration [μU/ml]
t: time[minute]

Here, $\alpha$ and $\beta$ are coefficient used for normalization

$$\alpha=1/\text{Average }\{\Sigma BG(t)\}$$

$$\beta=1/\text{Average }\{\Sigma PI(t)\}$$

The average of the formula shows average value with respect to all templates stored in the template database DB1.

Fig. 12 shows error summation (no normalization) of the OGTT time-series with respect to the template T1, and more specifically, Fig. 12(a) shows an error between the blood glucose level of Fig. 5(a) and the blood glucose level of Fig. 11(a), and Fig. 12(b) shows an error between the insulin of Fig. 5(b) and the insulin of Fig. 11(b).

Referring to the input data (data from 0 to 180 minutes every 10 minutes) of Fig. 9 and the template T1 of Fig. 11,

$$\Sigma|BG(t) - BGt(t)| = 29$$

$$\Sigma|PI(t) - PIt(t)| = 20$$

wherein, provided α= 0.00035, β = 0.00105

$$\text{error summation} = (0.00035 \times 29) + (0.00105 \times 20)$$
$$= 0.03115$$

Thus, the CPU 100a obtains an error summation for each template in the template database DB1, and determines a template having minimum error summation (similarity), that is, the CPU 100a determines the template which is the most approximate to the OGTT time-series data (Step S1-2).

[Acquisition of parameter set: Step 1-4]

Further, in step S1-3, the system SS acquires a parameter set corresponding to the template determined in step S1-2 from the template database DB1. That is, a parameter set PS#01 corresponding to the template T1 is obtained (see Fig. 10).

The following table 1 shows specific numeral values of the parameter values included in the parameter set PS#01 obtained as above.

[Table 1]

| Parameter set PS #01 with respect to template TI | | | |
|---|---|---|---|
| | Parameter | Value | Unit |
| Pancreas | h | 92.43 | [mg/dl] |
| | α | 0. 228 | [1/min] |
| | β | 0. 357 | [(μU/ml) · (dl/mg) · (1/min)] |
| | M | 1 | [1/min] |
| | X(0) | 336.4 | [μU/ml] |
| | Y (0) | 4.4 | [(μU/ml) · (1/min)] |
| Insulin kinetics | A1 | 0.025 | [1/min] |
| | A2 | 0.042 | [1/min] |
| | A3 | 0.435 | [1/min] |
| | A4 | 0.02 | [1/min] |
| | A5 | 0. 394 | [1/min] |
| | A6 | 0.142 | [1/min] |
| Peripheral tissues | Kb | 0.009 | [1/min] |
| | Kp | 5. 28E-05 | [(ml/μU) · (1/min)] |
| | u | 0. 6 | |

(continued)

| Parameter set PS #01 with respect to template TI | | | |
|---|---|---|---|
| | Parameter | Value | Unit |
| Liver | A7 | 0.47 | |
| | Kh | 0.0000462 | [(ml/μU) · (1/min) · (dl/kg)] |
| | b2 | 1. 1 | |
| | r | 0.98 | |
| | α2 | 0. 228 | |
| | $I_{4off}$ | 5 | [μU/ml] |

The method for generating the parameter set (biological model) is not limited to template matching as described above. For instance, the parameter set may be generated through genetic algorithm. That is, genetic algorithm may be applied of randomly producing an initial group of parameter set, and performing selection/chiasm/mutation process on the parameter set (individual) contained in the initial group to generate a new child group. Among the parameter sets generated through this genetic algorithm, the parameter set that outputs the pseudo-response close to the input biological response (test result) can be adopted.

Thus, the specific generating method of the biological model generating section is not particularly limited as long as the biological model that outputs pseudo-response simulating the input biological response can be generated.

[Pseudo-response acquiring unit (biological model calculating section)]

The system SS has a function of, when the parameter set PS#01 is provided to the biological model, performing a calculation based on the relevant biological model, and outputting pseudo-response information (time-series change in blood glucose level and insulin concentration) simulating the input OGTT time-series data (function as pseudo-response acquiring unit (biological model calculating section) of the system SS)

That is, the biological organs of the patient can be simulated based on the generated biological model in the system SS. This function is also realized by the pathological condition simulator/analysis program.

The generated parameter set is also used to acquire the pathological condition information (pathological condition feature information), which aspect will be hereinafter described.

[Function of application server S1]

Fig. 13 shows a screen of the present system SS generated by a screen control unit S1-A of the application server S1 of the server S and displayed on the display device of the client C.

The screen of Fig. 13 shows a starting screen immediately after the user such as doctor logs into the present system from the client C.

The screen of the present system SS is displayed on the Web browser C1 of the client C. In Fig. 13, a title bar C1-1, a tool bar C1-2, and a display region C1-3 of the Web browser C1 are shown. A screen to be displayed in the display region C1-3 of the browser C1 is generated in the server S. For the sake of simplification, only the display region C1-3 is shown in the figure showing the screen, and the illustrations of various bar C1-1, C1-2 displays of the Web browser will be omitted.

A patient search/list screen (hereinafter also referred to as "basic screen") W1 for searching the patient information and displaying the search result is displayed in the browser display region C1-3 of Fig. 13. The basic screen W1 is also a screen (route screen) that acts as a basis for transitioning to other screens W2, W3.

As also shown in Fig. 14, a medical examination main screen W2 (see Fig. 15) or a doctor screen for the doctors to browse through, and a patient screen W3 (see Fig. 16) for the patients to browse through can be transitioned from the basic screen W1. The user can transition to other two screens W1, W2, W3 from any one of the screens W1, W2, W3.

Each screen W1, W2, W3 includes operation screen regions W1A, W2A, W3A arranged with button displays for system operation such as screen transition operation etc., and main body screen regions W1B, W2B, W3B for displaying the content for each screen W1, W2, W3 and performing operation in each screen W1, W2, W3.

The operation screen regions W1A, W2A, W3A are the display content of the main body screen regions W1B, W2B, W3B, but are always displayed for each screen W1, W2, W3.

The operation screen regions W1A, W2A of the basic screen W1 and the doctor screen W2 are common, and include "patient search list" button 101, "main medical examination" button 102, and "patient view" button 103.

Such buttons 101, 102, 103 are selected by the mouse operation etc., to be able to transition from currently displayed screens W1, W2, W3 onto other screens W1, W2, W3.

The operation screen regions W1A, W2A of the basic screen W1 and the doctor screen W2 include a "notice" button 104 for displaying "notice" screen" from the system to the user, and "log out" button 105 for logging out from the system S.

The operation screen region W3A of the patient screen W3 includes "print" button 16 for executing a print process of printing the content of the screen displayed on the main body screen region B of the patient screen W3 by means of a printer, in addition to the transition buttons 101, 102 to other screens W1, W2. When the "print" button 106 is selected, the content of the current main body screen region B is printed. The screen content printed on the paper is handed to the patient, and this paper resource becomes the resource when the doctor gives an explanation to the patient, and the auxiliary resource for the patient to understand his/her condition.

The operation screen region W3A of the patient screen W3 also includes "log out" button 105. The operation screen region W3A of the patient screen W3 does not include the "notice" button 104, and the screen is simplified.

Fig. 14 shows an entire state transition of the system by the operation of the button displayed on the operation screen region of each screen W1, W2, W3 other than the screen transition. The reference numerals denoted on the arrow in Fig. 14 indicate the operation by the button (button of the operation screen region of the screens W1, W2, W3) having the corresponding reference numeral.

In the basic screen W1 and the doctor screen W2, the operation screen regions W1A, W2A are arranged at the upper part of the screens W1, W2 to which one tends to pay attention in order to ensure operability by the system user such as doctors or laboratory technicians.

The patient does not directly operate the system but simply looks at the screen, and thus the screen is simplified in the patient screen W3, and the operation screen region W3A is arranged at the lower part of the screen W3 to be easier for the patient.

Furthermore, in the operation screen region W3A of the patient screen W3, the button size is made smaller etc. so that buttons do not stand out more than the operation screen regions W1A, W2A of the basic screen W1 and the doctor screen W2.

[Details of patient search/list screen (basic screen) W1]

The details of the patient search/list screen W1 or the basic screen will be described below. The basic screen W1 has a function of searching the patient by inputting search conditions such as patient's name, and a function of displaying the patient list as a search result. Both functions may be configured by separate screens.

As shown in Fig. 13, the (main body screen region W1B) of the basic screen W1 includes a search operation part 110 for inputting the search condition etc., and a search result display part 130 for displaying the search result.

The basic screen W1 includes a new registration part 140 for newly registering the information of the patient. If the new registration part 140 is selected to perform the new registration process, the information on a new patient is registered in the database, and the relevant patient can be searched for.

The search operation part 110 includes input parts of various search conditions such as patient ID input part 111, carte ID input part 112, patient's name input part 113, patient name kana input part 114, sex input part 115, attending physician input part 116, date of birth input part 117, memo input part 118, elapsed number of days from last interview input part 119, test item name input part 120, and the like as search conditions.

In the present embodiment, one or more input conditions are input to the input part, and the search button 121 is selected so that the relevant search conditions are transmitted to the server S. When receiving the search conditions, the application server S1 of the server S searches the patient data table DB2 contained in the database of the database server S3. The content of the patient data table is as shown in Fig. 17(a). In the patient data table, the patient information corresponding to the search conditions is registered by patients.

When extracting the information of the patient that matches the search condition, the server S generates a basic screen in which the extracted information is displayed in a list in the search result display part 130 and transmits the same to the client C.

In the client C, the basic screen W1 transmitted from the server S is displayed. In the search result display part 130 of the basic screen W1 after the search, the patient search result list is displayed, as shown in Fig. 13. In the test result display part 130, the information on the patient extracted by the search condition (patient ID 131, patient name 132, sex 133, date of birth 134, attending physician 135, carte ID 136) are displayed. The patient list is normally organized in ascending order of the patient ID, but the ascending order or the descending order of the selected patient information (e.g., date of birth) can be reorganized by clicking each item 131 to 136 of the patient information.

The operation menu part 137 in which the operation can be selected is displayed for each patient in the search result display part 130. As shown in Fig. 18, if the operation menu part 137 for a certain patient is selected, "patient information edit" selecting part 151, "test data input" selecting part 152, "main medical examination" selecting part 153, and "patient view" selecting part 154 are displayed as selectable operation menus.

Since the operation menu part 137 is displayed for every patient, the selection of the operation menu and selection of the patient can be performed at the same time.

When the user such as the doctor selects the "patient information edit" selecting part 151, the server S references the patient data table DB2 (see Fig. 17(a)) of the database with the patient ID of the selected patient as the key information, and the patient information to be edited is extracted. The server S then generates a "patient information edit" screen on which the information of the patient (content of patient data table) is edited, and displays the same to the client C (see Fig. 19). After the editing of the patient information is terminated, the server S updates the content of the patient data table DB2 for the relevant patient. The screen then returns to the original basic screen W1.

When the user such as the doctor selects the "test data input" selecting part 152, the server S generates a "test data input screen" on which the test data is input, and displays the same to the client C (see Figs. 21 and 22). After the input of the test data is terminated, the screen returns to the original basic screen W1.

The test data input screen is configured such that the basic test data screen (Fig. 20) for inputting the basic test values such as weight, the OGTT data screen (Fig. 21) for inputting the OGTT data, the target value screen (Fig. 22) for inputting the target value of the basic test item can be switch displayed by tab form.

In the basic test data screen of Fig. 20, the test result of each test item can be input for every test date. The data input in the basic test data screen is registered in the basic test result data table DB3 of the database as test result for every test date along with the patient ID.

In the OGTT data screen of Fig. 21, the OGTT glucose data (blood glucose level change data) and the OGTT insulin data (blood insulin concentration change data) are input as the OGTT time series data for every test date. The data input in the OGTT data screen are registered in the OGTT data table DB4 of the database as test data for every test date along with the patient ID (see Fig. 17(c)).

In the target value screen of Fig. 22, the target value for the basic test items such as weight can be input. The data input in the target value screen are registered in the target value data table DB5 along with the patient ID (see Fig. 17(d)).

When new registration or correction of the OGTT data is performed in the OGTT data screen of FIG. 21, the simulator/analysis server S2 performs simulation and pathological condition analysis by using the OGTT data, and acquires the pathological condition information (pathological condition feature information) showing the features of the pathological condition. The acquired pathological condition feature information is registered in the OGTT data table DB4 of the database in association with the OGTT data. The details of the pathological condition analysis will be hereinafter described.

Since the weight information (one of the basic test items) of the patient is also necessary in simulation and pathological condition analysis, the weight of the patient at the test date of the most recent basic test is acquired from the basic test result data table DB.

Thus, since the basic test data are also used in simulation and pathological condition analysis, re-execution of the simulation and re-analysis of the pathological condition are performed in the system SS not limited to when new registration and correction of the OGTT data are performed in the OGTT data screen of Fig. 21, but also when registration and correction of the basic test data are performed in the basic test data input screen of Fig. 20. The re-acquired pathological condition feature information is registered in the OGTT data table DB4 of the database in association with the OGTT data used in the simulation.

Returning to Fig. 18, when the user such as the doctor selects the "main medical examination" selecting part 153 in basic screen W1, the server S generates "main medical examination" screen (doctor screen) W2 displaying information on the selected patient, and displays the same to the client C (see Fig. 15).

The doctor screen W2 displays information on the "patient" selected in the basic screen W1, wherein the server S references the data tables DB2 to DB7 in the database with the patient ID of the selected patient as the key, extracts the information on the relevant patient, and generates the doctor screen W2 for displaying the information on the patient to support the diagnosis made by the doctor.

When the user such as the doctor selects "patient view" selecting part 154 in the basic screen W1, the server S generates "patient view" screen (doctor screen) W3) displaying information on the selected patient, and displays the same to the client C (see Fig. 16).

The patient screen W3 displays information on the "patient" selected in the basic screen W1, and the server S references the data tables DB2 to DB7 in the database with the patient ID of the selected patient as the key, extracts the information on the relevant patient, and generates the patient screen W2 for displaying the information that can be browsed by the patient.

[Supplementary explanation on screen switching function (screen switching unit S1-B)]

Here, the difference between the screen transition by the operation of the "main medical examination" selecting part 153 and the "patient view" selecting part 154 of Fig. 18, and the screen transition by the operations of the "main medical examination" button 102 and the "patient view" button 103 of the operation screen regions W1A, W2A, W3A of Figs. 13, 15, and 16 will be described.

Both screen transitions are the same in that the screen is switched to the main medical examination screen W2 or the doctor screen, or the patient view W3 screen or the patient screen. That is, in either case, the screen switching unit S1-B of the server S accepts the input for performing display switching from the client C and switches the screen.

The former is used when displaying the main medical examination screen or the patient view of the selected patient while selecting the patient. That is, in order to switch to the display of "different patient", the user needs to retum to the basic screen W1, and perform the former screen transition.

The latter is used to switch the main medical examination screen or the patient view for "the same patient". In the latter case, the user does not need to return to the basic screen W1 to switch the screens, and the switch can be rapidly carried out with the operations of the operation screen regions W2A, W3A of each screen W2, W3.

Therefore, the doctor makes a diagnosis while browsing the doctor screen W2, and at that moment, directly (without passing through the basic screen W1) transition to the patient screen W3 from the doctor screen W2 when indicating the necessary information to the patient. The reverse is also possible.

[Details of main medical examination (doctor screen) W2]

As apparent from the doctor screen W2 shown in Fig. 15 and the doctor screen W2 shown in Fig. 23, the main body screen region W2B of the doctor screen W2 includes a fixed display part 161 position fixedly displayed at the upper part of the main body screen region W2B. A multi-screen region (multi-window region) in which a plurality of position movably displayed screen regions (windows) are simultaneously displayed is formed at the region on the lower side of the fixed display part 161 of the main body screen region W2B.

The fixed display part 161 displays patient information (information of patient data table DB2) such as name of patient, date of birth, patient ID, and the like, and furthermore, is arranged with "edit patient information" button 161a for displaying the patient information edit screen for editing the information of the patient and "reset arrangement" button 161b for returning the arrangement (position, size) of a plurality of screen regions to an initial state.

In the multi-screen region of the present embodiment, four screen regions (windows) of the basic test data screen region (basic test data window) 162, the OGTT data screen region (OGTT data window) 163, the prescription screen region (prescription window) 164, and the finding screen region (finding window) 165 are simultaneously displayed.

Each screen region 162 to 165 is position adjustable within the multi-screen region of the doctor screen W2 by the operation of the mouse etc., and the size thereof is also adjustable.

Among the screen regions 162 to 165, the basic test data screen region (screen region for displaying the related information on the patient) 162 and the OGTT data screen region (screen region for displaying the pathological condition information of the patient) 163 are particularly important to support the diagnosis by the doctor. The basic test data screen region 162 shows the test result obtained by the actual test (basic test), and the OGTT data screen region 163 shows the actual OGTT result and the pathological condition information or the result of the simulation analysis.

The basic test screen region 162 is generated by the server S based on the information (test result) stored in the basic test result data table DB3 shown in Fig. 17(b). The server S searches the basic test result data table with the patient ID of the selected patient as the key, and generates the test result information on the most recent test date in the basic test screen region 162 as the test result display part 162a.

In the basic test screen region 162, each test item name in the basic test result display part 162 can be selected by mouse operation (double click etc.), and the time-series graph 162b on the selected item is displayed at the position in the basic test screen region 162 (right side of test result display part 162a). If the target value is set for the selected test item, not only the test value Tst but also the target value Tr is displayed in the time-series graph 162b.

The time-series graph 162b can be selected up to a maximum of four in the basic test screen region 162 of the doctor screen W2. Four time-series graphs 162b are shown in Fig. 15, and one time-series graph 162b is shown in Fig. 23.

The test result displayed in the test result display part 162a can be changed to a test result of a different test date. Thus, the basic test screen region 162 includes "another test date" button 162c for selecting an arbitrary test date and displaying the test result of the relevant test date, "previous test date" button 162d for displaying the test result of the previous test date, and "next test date" button 162e for displaying the test result of the next test date.

The OGTT data screen region (screen region for displaying pathological condition information of the patient) 163 shown in Fig. 15 is generated by the server S based on the information (test data and analysis result) stored in the OGTT data table DB4 shown in Fig. 17(c). The server S searches the OGTT data table DB4 with the patient ID of the selected patient as the key, acquires the test data (OGTT time-series data) and the analysis result (pathological condition information) on the most recent test date, and generates the time-series graph 163a of the OGTT test data and the radar chart 163b of the analysis result in the OGTT screen region 163.

A time-series change 163a-1 of the blood glucose level in the actual OGTT test result and a time-series change 163a-2 of the insulin concentration in the actual OGTT test result are displayed in the time-series graph 163a.

The radar chart 163b shows the result of performing the pathological condition analysis. The details of the process of performing pathological condition analysis from simulation based on the biological model and acquiring the pathological condition information (pathological condition feature information) will be described below.

[Pathological condition simulation analysis (pathological condition information acquisition)]

In the above described simulation using the biological model, the parameter set configuring the biological model capable of outputting the reproduction value (pseudo-response information) simulating the actual OGTT test result (biological response information) is obtained through calculation. The system SS obtains the reproduction value (pseudo-response information) of the OGTT test result as the output value of the biological model applied with such parameter set (pseudo-response acquiring function of the system SS).

The simulator/analysis server S2 obtains the pathological condition information (pathological condition feature information) indicating the feature of the pathological condition of the patient based on the generated biological model (parameter set thereof) (pathological condition feature information acquiring function of the system SS).

In the present embodiment, P1: fasting blood glucose, P2: basic secretion, P3: additional secretion, P4: secretion sensitivity, P5: glucose regeneration suppression, P6: glucose disposal ability, and P7: processing sensitivity are adopted as indices of the pathological condition feature.

Such indices are adopted as satisfactorily representing the features of the pathological condition, and in particular, biological functions that can be improved by treatment are adopted. The indices of the pathological condition feature are not limited to the above.

Here, P1: fasting blood glucose is calculated from the blood glucose level BG (t=0) which is a variable of the biological model. P2: basic secretion is calculated from the fasting insulin $I_1$ (t=0) which is a variable of the biological model. P3: additional secretion is calculated from an integrated value of $I_1$ (t=). P4: secretion sensitivity is calculated from the sensitivity $\beta$ with respect to glucose stimulation or the parameter of the biological model. P5: glucose regeneration suppression is calculated from the glucose release HGTP(t) which is a variable of the biological model. P6: glucose disposal ability is calculated from the net glucose SGO(t) and the blood glucose level BG(t) from the liver, which are variables of the biological model. P7: processing sensitivity is calculated from the insulin dependent glucose consumption rate Kp in the peripheral tissue per unit insulin or unit glucose, which is a parameter of the biological model.

Therefore, since the biological model is configured by a mathematical model having parameters (include variables) indicating the characteristics of the biological organs, the parameter values of the biological model indicate values related to the pathological condition. Therefore, the pathological condition information (pathological condition feature information) indicating the features of the pathological condition can be calculated based on such parameters.

The calculated pathological condition information is registered in the OGTT data table DB4 of the database as analysis result, and used to create a radar chart 163b.

In the radar chart 163b of Fig. 15, the values for every index of the pathological condition feature information are scored and displayed, so that good and bad of the value of each index having different units and numeral widths can be compared.

The doctor looks at the radar chart 163b to diagnose the pathological condition of the patient. For instance, if the radar chart 163b shows that the glucose disposal ability and the processing sensitivity related to the glucose disposal ability at the peripherals are low, the doctor looking at the radar chart can easily determine that the treatment method that improves the glucose disposal ability at the peripherals is effective.

Furthermore, since the actual OGTT test result 163a and the pathological condition information 163b are simultaneously displayed, the doctors can compare both displays and learn about the relation of the graph shape of the OGTT test result and the pathological condition. Therefore, an effective learning effect for understanding the pathological condition from the OGTT test result can be expected by building up experiences by actually using the system SS.

Furthermore, since the basic test data screen region 162 and.the pathological condition information 163b are simultaneously displayed in the present embodiment, the doctor can also build up experience of understanding the pathological condition even in relation to the basic test result.

Thus, in the doctor screen W2, the screen region 163 for displaying the pathological condition information of the patient and the screen region 162 for displaying the related information (in particular, basic test information) related to the patient are simultaneously displayed, thereby supporting the doctor to make an accurate diagnosis based on great amount of information. The doctor can further build up experience since great amount of information are displayed.

Moreover, arbitrary values can be input instead of the actual test result (biological response information) in the input screen of the OGTT test result (biological response). The output of the test result reproduction value (pseudo-response information) on the arbitrary value and the pathological condition feature information can be obtained. Therefore, the doctor can input an arbitrary test result value (biological response information) and see what pathological condition is obtained in the relevant case. Thus, the system can be used for training purpose by inputting an appropriate test result even if the actual data of the patient are not provided, and checking what kind of output is obtained.

That is, the present system SS can be used for training purpose for non-specialists or doctors with little experience to learn.

A "pathological condition review/recommended treatment/term explanation" column (pathological condition review display part) 163c is arranged in the OGTT data screen region 163, wherein text explanation (pathological condition review) such as review of the pathological condition, recommended treatment, term explanation etc. is displayed. Since the "pathological condition review/recommended treatment/term explanation" column 163c is arranged, the pathological condition feature information displayed on the radar chart 132 can be easily understood, and the doctor can more accurately understand the pathological condition. Information useful for determining the treatment method etc. is also obtained.

The pathological condition review text displayed on the pathological condition review display part 163c is registered in the database in advance, the pathological condition review text corresponding to the generated biological model (parameter thereof) is selected by the system, and the selected text is displayed on the pathological condition review display part 163c.

In the OGTT data screen region 163, a special instruction display part 163d is also arranged, so that when there is a special instruction on the pathological condition information obtained by analysis, such special instruction is displayed on the special instruction display part 163.

The data displayed on the time-series graph 163a and the radar chart 163b of the OGTT data screen region 163 can be changed to those of a different test date. Thus, the basic test screen region 162 includes "another test date" button 163e for selecting an arbitrary test date and displaying the test result of the relevant test date and the pathological condition information based on the relevant test result, "previous test date" button 163f for displaying the test result etc. of the previous test date, and "next test date" button 163g for displaying the test result etc. of the next test date.

An overlay display for displaying the data of different test dates in an overlapping manner is possible in the time-series graph 163a and the radar chart 163b of the OGTT data screen region 163. The check box 163h of the overlay display of the OGTT data screen region 163 is checked, the "select date" or the date selecting button is selected, and the test date of the data desired to be displayed in an overlapping manner on the current time-series graph 163a and the radar chart 163b is selected, so that the time-series graph 163a and the radar chart 163b of both test dates are displayed in an overlapping manner.

Therefore, the doctor can simultaneously compare the OGTT data and the pathological condition information of different dates, and make a diagnosis based on temporal changes of the OGTT data and the pathological condition information.

In the OGTT data screen region 163, the OGTT test data and the analysis result (pathological condition information) may be displayed as it is as numerical information instead of being illustrated as the time-series graph 163a and the radar 163b. In the screens shown in Figs. 15 and 23, a display in which the OGTT test data and the analysis result (pathological condition information) are shown in a form of a diagram, and a display in numerical data can be switched by a tab form, wherein the numerical data of the OGTT test data and the analysis result (pathological condition information) can be displayed in the OGTT data screen region 163 by selecting the "numerical data" tab 163j.

As shown in Fig. 23, in the doctor screen W2, the prescription screen region (prescription window) 164 and the finding screen region (finding window) 165 are also displayed.

When the doctor understands the pathological condition, determines the treatment, and decides on the prescription content, the prescription content can be registered for every medical examination date in the prescription screen region 164. The prescription content is registered for every medical examination date in the prescription data table DB6 shown in Fig. 17(e).

The pathological condition understood by the doctor can be registered in the finding screen region 165 as finding of the doctor. The finding content is registered for every medical examination date in the finding data table DB7 shown in Fig. 17(f).

Since the findings and the prescription content of the doctor can be registered, the present system SS also has a function serving as an electronic carte, thereby enhancing convenience.

Furthermore, since registration task of prescription, finding, or the like is possible in the prescription screen region (prescription window) 164 or the finding screen region (finding window) 165 simultaneously displayed with the pathological condition information 163b and the like on the doctor screen W2, high convenience is achieved also in this point.

The prescription screen region 164 and the finding screen region 165 are both registered in the data tables DB6, DB7, similar to the basic test data screen region 162 and the OGTT data screen region. The prescription content or the finding content on another medical examination date can also be displayed.

[Details of patient view (patient screen) W3]

Figs. 24 to 27 show the patient screen W3 in addition to Fig. 16.

The main body screen region W3B of the patient screen W3 includes the fixed display part 181 position fixedly displayed at the upper part of the main body screen region W3B.

The region on the lower side of the fixed display part 181 of the main body screen region W3B is formed as a tab switching screen region 182 capable of switching a plurality of screen regions by tab switching.

The fixed display part 181 displays patient information (information of patient data table DB2) such as name of patient, date of birth, attending physician, and the like. In the fixed display part 181 of the patient screen W3, as compared with the fixed display part 161 of the doctor screen W2, information not necessarily required for the patient are omitted, for example, patient ID is not displayed, whereby information amount is reduced, and a simple and viewable display is obtained. The fixed display part 181 of the patient screen W3 has larger characters, and thus is an easily viewable display. The fixed display part 181 of the patient screen W3 is not arranged with the buttons 161a, 161b in the fixed display part 161 of the doctor screen W, and thus is a simple and easily viewable display.

The tab switching screen region 182 includes four screen switching tabs 182a, 182b, 182c, and 182d. Among the tabs, the "basic test data" tab 182a displays the basic test data screen region 192, the "OGTT data" tab 182b displays the OGTT data screen region 193, the "prescription/finding" tab 182c displays the prescription/finding screen region 194, and "past prescription list" tab 182d displays the past prescription list screen region 195.

Figs. 16 and 24 show the basic test data screen region 192 of the patient screen W3. The basic test data screen region 192 is generated from the basic test result data table DB3 with the patient ID as the key.

The basic test data screen region 192 cannot change position and size, but is formed over substantially the entire tab switching screen region 182, and is formed larger than the basic test data screen region 162 (size of initial state: see Figs. 2 and 3) of the doctor screen W2.

Similar to the basic test data screen region 162 of the doctor screen W2, the test result information on the most recent test date is displayed as test result display part 192a on the basic test data screen region 192 of the patient screen W3. The test result display part 192a is formed larger than the test result display part 162a of the doctor screen W2, and displays greater amount of information. Specifically, target values set for each test item are contained in the test result display part 192a of the patient screen W3. The target value is obtained by referencing the target value data table DB5 (see Fig. 17(d)) with the patient ID as the key.

In the basic test screen region 192, each test item name in the basic test result display part 192 can be selected by mouse operation (double click etc.), and the time-series graph 192b for the selected item is displayed at the position in the basic test data screen region 192 (right side of test result display part 192a).

Up to four time-series graphs 192b can be selected in the basic test data screen region 192 of the patient screen W3.

The test result displayed in the test result display part 192a can be changed to a test result of a different test date. Thus, the basic test screen region 192 includes "another test date" button 192c for selecting an arbitrary test date and displaying the test result of the relevant test date. The test result display part 192a is arranged with "display/not display comparison column" switching button 192d for displaying the basic test data of another test date as a comparison column or not displaying the relevant display.

As described above, other screen regions 193 to 195 are displayed when the basic test data screen region (test result screen) 192 is displayed in the patient screen W3. Therefore, the patient screen W3 in which the basic test data screen region 192 of the patient screen W3 is selectively displayed is a test result screen for displaying the result information of a medical test of the patient without involving display of the pathological condition information.

Fig. 24 shows the OGTT data screen region 193 of the patient screen w3. The OGTT data screen region 193 is generated based on the OGTT data table DB4 with the patient ID as the key.

The OGTT data screen region 193 also can not change the position or the size, but is formed over substantially the entire tab switching screen region 182, and is formed larger than the OGTT data screen region 162 of the doctor screen W2.

In the OGTT data screen region 193 of the patient screen W3 as well, the time-series graph 193a of the OGTT test data and the radar chart 193b of the analysis result (pathological condition information) can be displayed. Furthermore, in the OGTT data screen region 193 of the patient screen W3, the numerical data 193c of the OGTT test data that could not be displayed unless switched with the tab is simultaneously displayed. The numerical data could not be displayed unless switched with the tab in the doctor screen W2, but is displayed with the graph in the patient screen W2.

Furthermore, in the OGTT data screen region 193 of the patient screen W3, information that are not directly necessary for the patient such as "pathological condition review/recommended treatment/term explanation" column 163c and the special instruction 163d in the OGTT data screen region 162 of the doctor screen W2 are not displayed.

In the OGTT data screen region 193 of the patient screen W3, information 193a to 193c on a plurality of test dates can be simultaneously displayed, and the patient can simultaneously compare information on a plurality of test dates.

Therefore, in the patient screen W3, information not necessary for the patient out of the information displayed in the doctor screen W2 are omitted, and information useful for the patient are displayed in detail in a large space.

The information of the basic test data screen region 192 and the OGTT data screen region 193 are information of different tests for the user, and the necessity to simultaneously view them is low, and thus the convenience of the patient does not lower even if the screen regions 192, 193 are selectively displayed.

Fig. 26 shows the prescription/finding screen region 194 of the patient screen W3. The prescription/finding screen region 194 is generated from the prescription data table DB6 and the finding data table DB7 with the patient ID as the key, and the collection of the display content of the prescription screen region 164 and the display content of the finding screen region 165 of the doctor screen W2 is displayed.

Furthermore, if the prescription/finding screen region 194 is selectively displayed, "G" selecting part 107 is displayed as the image selecting part on the operation screen region W3A. The image selecting part 107 is provided for the user such as the doctor to switch and select the image 194g displayed on the prescription/finding screen region 194.

Fig. 27 shows the "past prescription list" screen region 195 of the patient screen W3. The "past prescription list" screen region 195 is generated from the prescription data table DB6 with the patient ID as the key, and a list of medicines prescribed in a predetermined period in the past is displayed.

Each screen region 192 and 193 of the patient screen W3 described above is printed by selecting the "print" button 106. As opposed to each screen region 162 to 165 of the doctor screen W2, since switching of the display content is barely performed in each screen region 192 and 193 of the patient screen W3, all the necessary information can be printed even if the content of the screen is printed as it is, and a printout easily viewable for the patient can be obtained.

The present invention is not limited to the above embodiment, and various modifications may be made. For instance, the display format of the pathological condition information is not limited to a radar chart and may be in other display formats. The type and number of pathological condition information to be displayed are not particularly limited, and three indices of hepatic glucose metabolism ability, insulin secretion ability, and peripheral insulin sensitivity may be displayed as pathological condition information.

The data table is shown in plurals in Fig. 17, but such data tables may be integrated into one table.

In the above embodiment, the data of the most recent test date of the patient is displayed when the screen is switched between the doctor screen W2 and the patient screen W3, but in the doctor screen W2 or the patient screen W3, when switched to another screen W2, W3 after selecting another test date as the test date, the data of another test date may still be continuously displayed in the switched screen W2, W3.

## Claims

1. A medical diagnosis support computer system for supporting diagnosis of a patient by a doctor, the system comprising:

   a display device; and
   a controller in communication with the display device, the controller comprising a processor configured to build a biological model representing biological functions of the patient on a computer, generate pathological condition information of the patient based on the biological model, display a doctor screen for the doctor and a patient screen for the patient in a switchable manner on the display device, and accept an input for switching the display between the doctor screen and the patient screen; wherein
   the doctor screen is configured to simultaneously display a screen region for displaying the pathological condition information of the patient and a screen region for displaying one or more related information related to the patient; and
   the patient screen is configured to selectively display a display content between the pathological condition information of the patient and one or more related information related to the patient.

2. The medical diagnosis support computer system according to claim 1, wherein the related information comprises result information of a medical test of the patient.

3. The medical diagnosis support computer system according to claim 1 or 2, wherein the related information comprises finding information which is a result of a medical examination by the doctor.

4. The medical diagnosis support computer system according to any one of claims 1 to 3, wherein the related information comprises prescription information indicating administration state of medicines to the patient.

5. The medical diagnosis support computer system according to any one of claims 1 to 4, wherein the screen region for selectively displaying the pathological condition information or the related information in the patient screen is larger than the screen region for displaying the pathological condition information and the screen region for displaying the related information in the doctor screen.

6. The medical diagnosis support computer system according to any one of claims 1 to 5, wherein the pathological condition information comprises pathological condition information of diabetes obtained through simulation analysis using the biological model.

7. The medical diagnosis support computer system according to any one of claims 1 to 6, wherein display of the pathological condition information comprises a radar chart showing pathological condition information obtained through simulation analysis using the biological model.

8. The medical diagnosis support computer system according to any one of claims 1 to 7, wherein

the doctor screen displays a doctor/patient switch receiving unit for receiving display switching to the patient screen;
the patient screen displays a patient/doctor switch receiving unit for receiving display switching to the doctor screen; and
the processor switches the screen on the same patient when receiving an input for switching the display.

9. The medical diagnosis support computer system according to any one of claims 1 to 8, wherein the processor further displays a basic screen on the display device for displaying a basic/doctor switch receiving unit for receiving display switching to the doctor screen and a basic/patient switch receiving unit for receiving display switching to the patient screen.

10. A computer program product, comprising:

a computer readable medium; and
instructions, on the computer readable medium, adapted to enable a general purpose computer to perform operations, comprising:

building a biological model representing biological functions of the patient on a computer;
generating pathological condition information of the patient based on the biological model;
displaying a doctor screen for the doctor and a patient screen for the patient in a switchable manner on the display device; and
receiving an input for switching the display between the doctor screen and the patient screen; wherein
the doctor screen is configured to simultaneously display a screen region for displaying the pathological condition information of the patient and a screen region for displaying one or more related information related to the patient; and
the patient screen is configured to selectively display a display content between the pathological condition information of the patient and one or more related information related to the patient.

11. A medical diagnosis support computer system for supporting diagnosis of a patient by a doctor, the system comprising:

a display device; and
a controller in communication with the display device, the controller comprising a processor configured to build a biological model representing biological functions of the patient on a computer, generate pathological condition information of the patient based on the biological model, display a doctor screen for the doctor and a patient screen for the patient in a switchable manner on the display device, and receive an input for switching the display between the doctor screen and the patient screen; wherein
the doctor screen is configured to simultaneously display a screen region for displaying the pathological condition information of the patient and a screen region for displaying one or more related information related to the patient; and
the patient screen comprises a test result screen for displaying result information of a medical test of the patient without display of the pathological condition information.

**12.** The medical diagnosis support computer system according to claim 11, wherein the screen region of the test result screen in the patient screen is larger than the screen region for displaying the related information in the doctor screen.

**13.** A computer program product, comprising:

a computer readable medium; and
instructions, on the computer readable medium, adapted to enable a general purpose computer to perform operations, comprising:

building a biological model representing biological functions of the patient on a computer;
generating pathological condition information of the patient based on the biological model;
displaying a doctor screen for the doctor and a patient screen for the patient in a switchable manner on the display device; and
receiving an input for switching the display between the doctor screen and the patient screen; wherein
the doctor screen is configured to simultaneously display a screen region for displaying the pathological condition information of the patient and a screen region for displaying one or more related information related to the patient; and
the patient screen comprises a test result screen for displaying result information of a medical test of the patient without display of the pathological condition information.

**14.** A server computer for displaying a screen for supporting a diagnosis of a patient by a doctor on a display device of a terminal device connected by way of a network, the server computer comprising:

a controller in communication with the terminal device, the controller comprising a processor configured to display a doctor screen for the doctor and a patient screen for the patient in a switchable manner on the display device of the terminal device, and switch the display between the doctor screen and the patient screen when an input for switching between the doctor screen and the patient screen is made at the terminal device; wherein
the doctor screen is configured to simultaneously display a screen region for displaying pathological condition information of the patient generated based on a biological model built on a computer to represent biological functions of the patient and a screen region for displaying one or more related information related to the patient; and
the patient screen is configured to selectively display a display content between the pathological condition information of the patient and one or more related information related to the patient.

**15.** A server computer for displaying a screen for supporting a diagnosis of a patient by a doctor on a display device of a terminal device connected by way of a network, the server computer comprising:

a controller in communication with the terminal device, the controller comprising a processor configured to display a doctor screen for the doctor and a patient screen for the patient in a switchable manner on the display device of the terminal device, and switch the display between the doctor screen and the patient screen when an input for switching between the doctor screen and the patient screen is made at the terminal device; wherein
the doctor screen is configured to simultaneously display a screen region for displaying pathological condition information of the patient generated based on a biological model built on a computer to represent biological functions of the patient and a screen region for displaying one or more related information related to the patient; and
the patient screen includes a test result screen for displaying result information of a medical test of the patient without display of the pathological condition information.

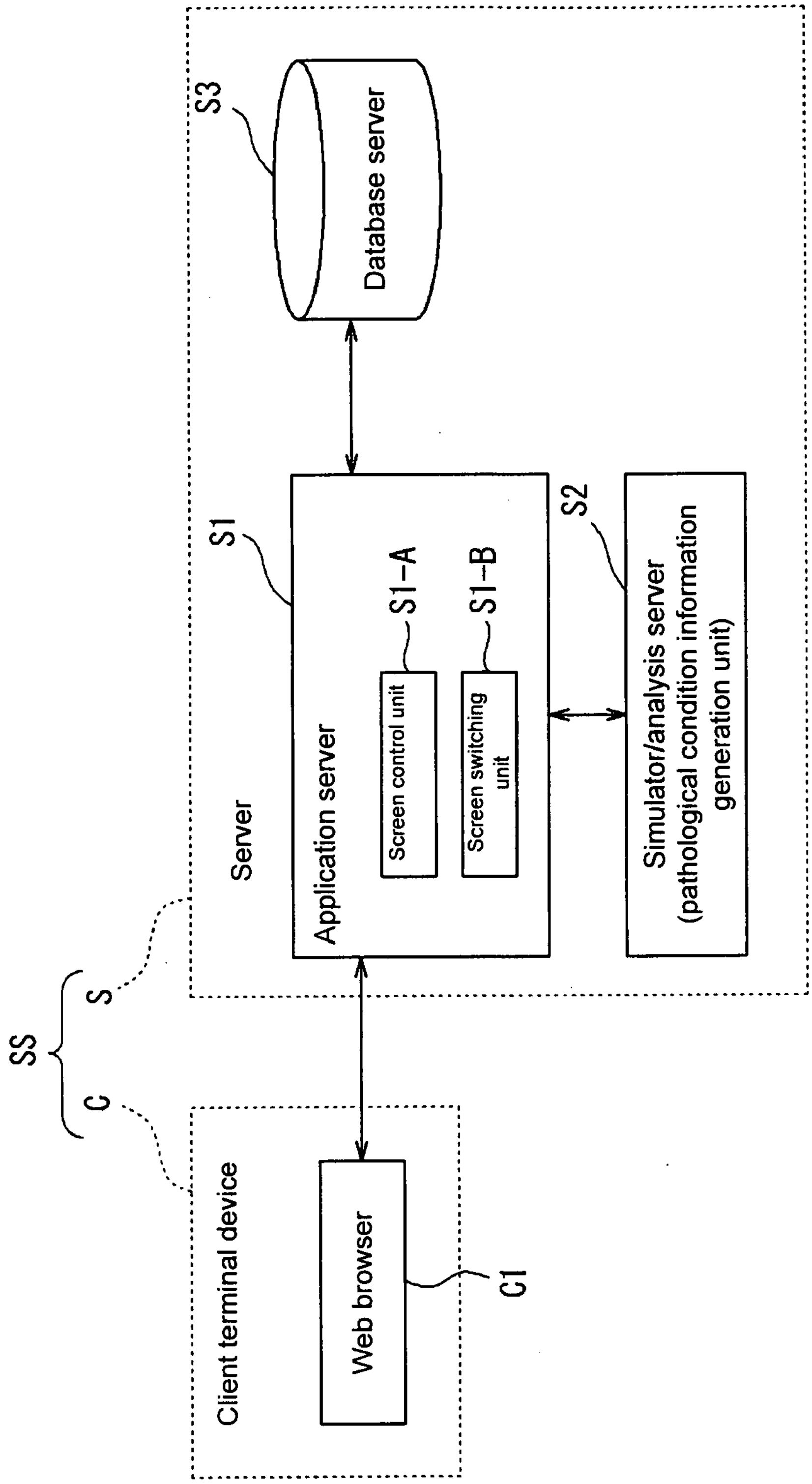
Database server
S3
Server
Application server
S1
Screen control unit
S1-A
Screen switching unit
S1-B
Simulator/analysis server (pathological condition information generation unit)
S2
SS
S
C
Client terminal device
Web browser
C1

FIG. 1

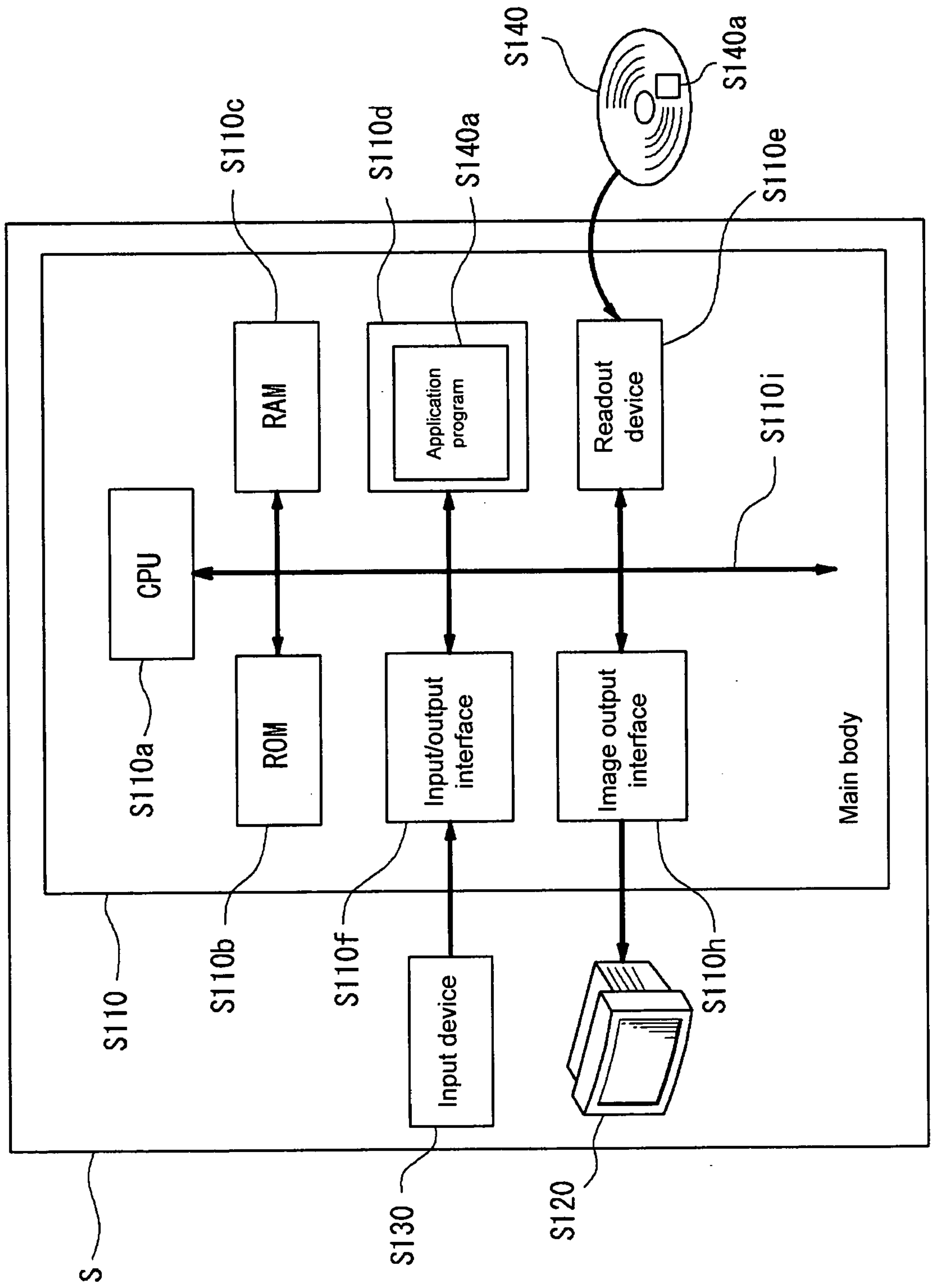
S
S110
S110a
CPU
S110b
ROM
S110c
RAM
S110d
S140a
Application program
S110e
Readout device
S110f
Input/output interface
S110h
Image output interface
S110i
Main body
S120
S130
Input device
S140
S140a

FIG. 2

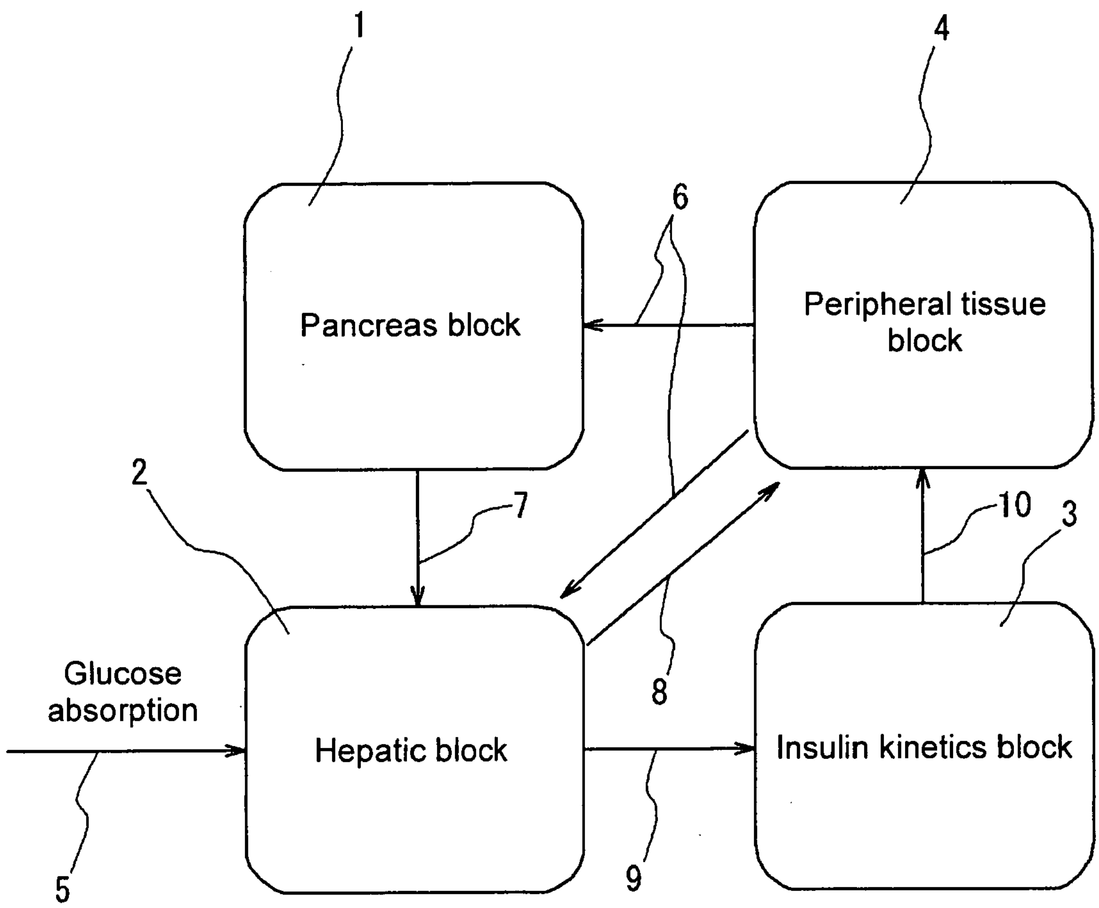
1
4
6
Pancreas block
Peripheral tissue block
2
7
10
3
Glucose absorption
Hepatic block
8
Insulin kinetics block
5
9

FIG. 3

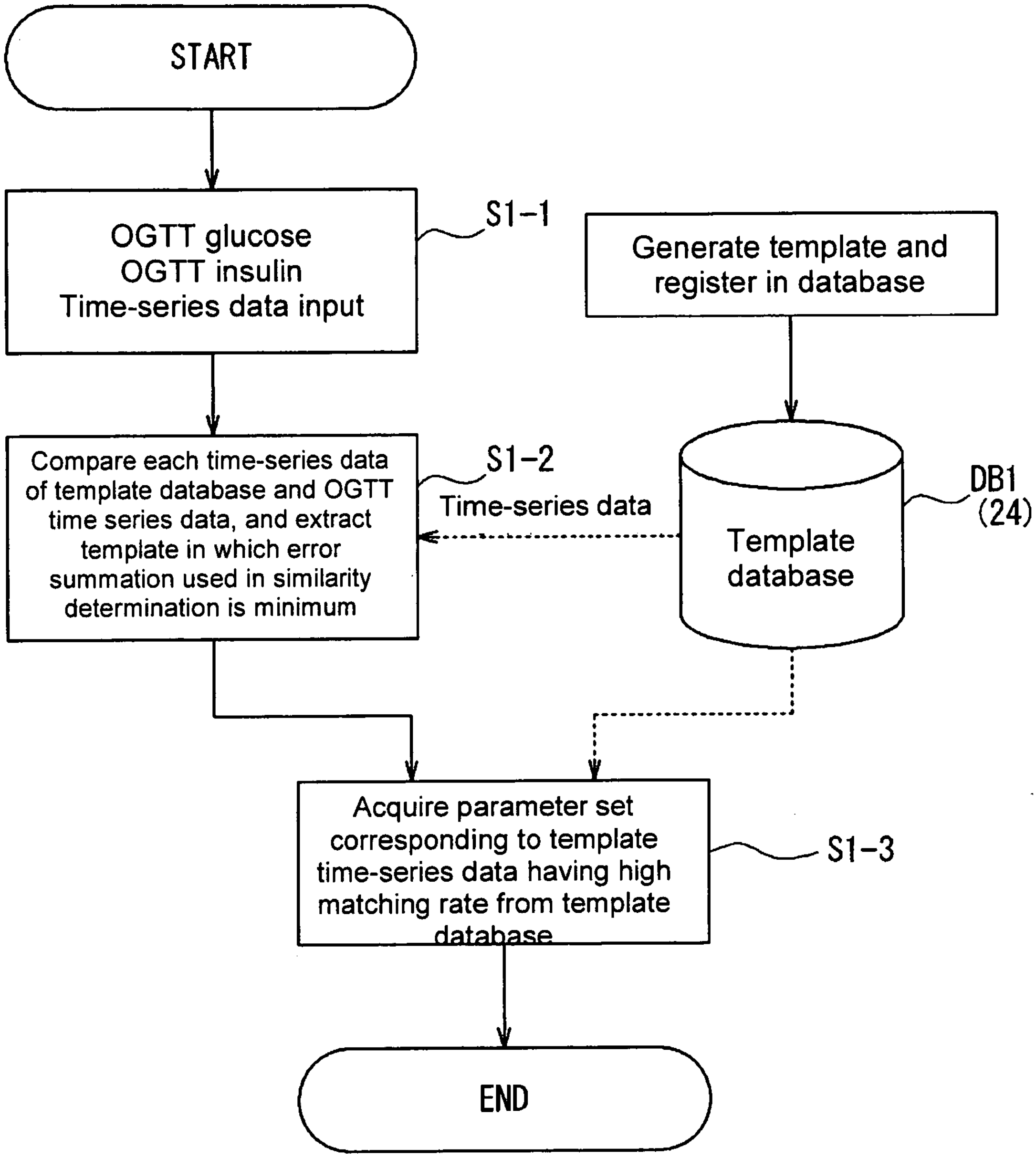
START
OGTT glucose
OGTT insulin
Time-series data input
S1-1
Generate template and
register in database
Compare each time-series data
of template database and OGTT
time series data, and extract
template in which error
summation used in similarity
determination is minimum
S1-2
Time-series data
Template
database
DB1
(24)
Acquire parameter set
corresponding to template
time-series data having high
matching rate from template
database
S1-3
END

FIG. 4

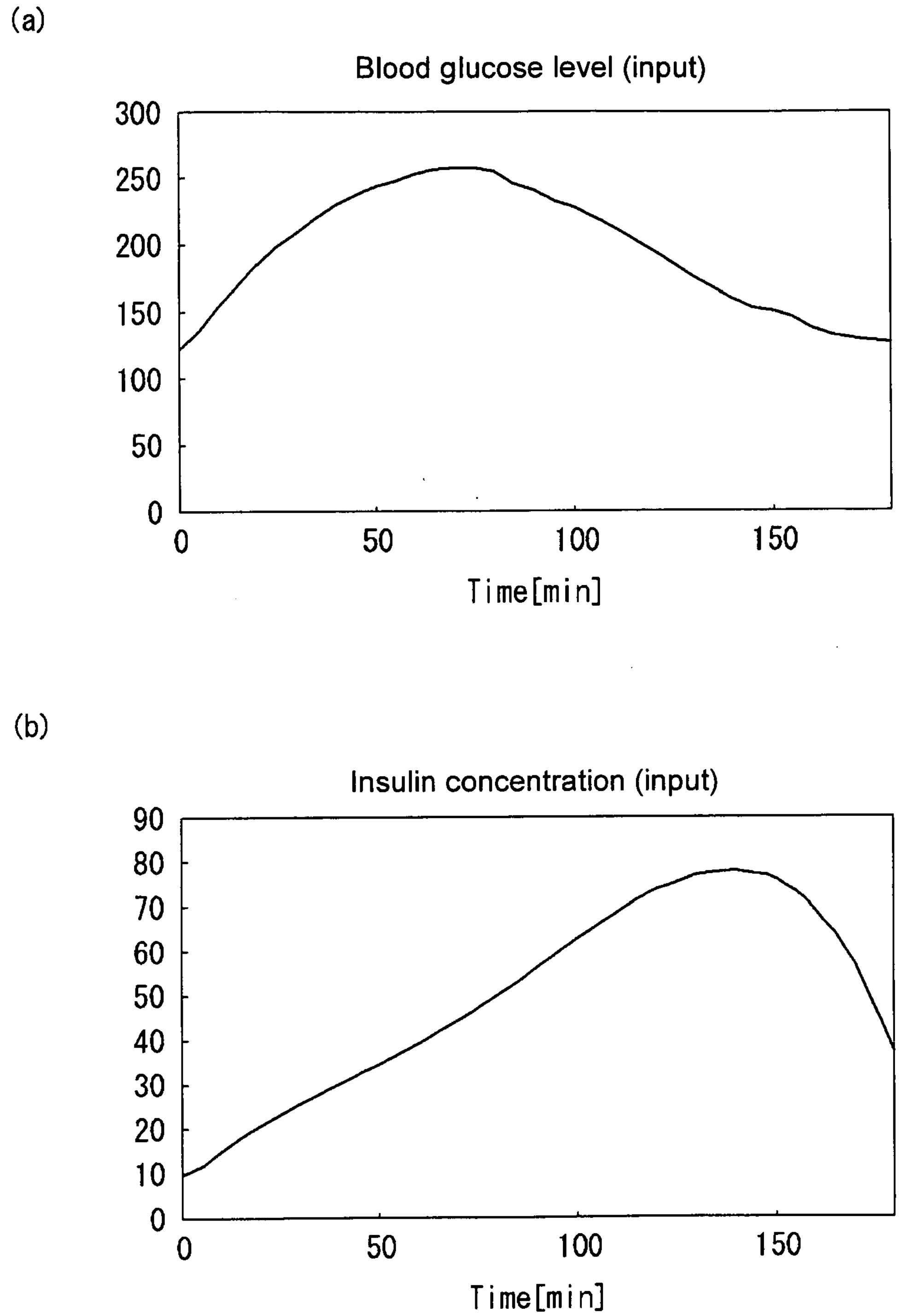
(a)
Blood glucose level (input)
300
250
200
150
100
50
0
0
50
100
150
Time[min]
(b)
Insulin concentration (input)
90
80
70
60
50
40
30
20
10
0
0
50
100
150
Time[min]

FIG. 5

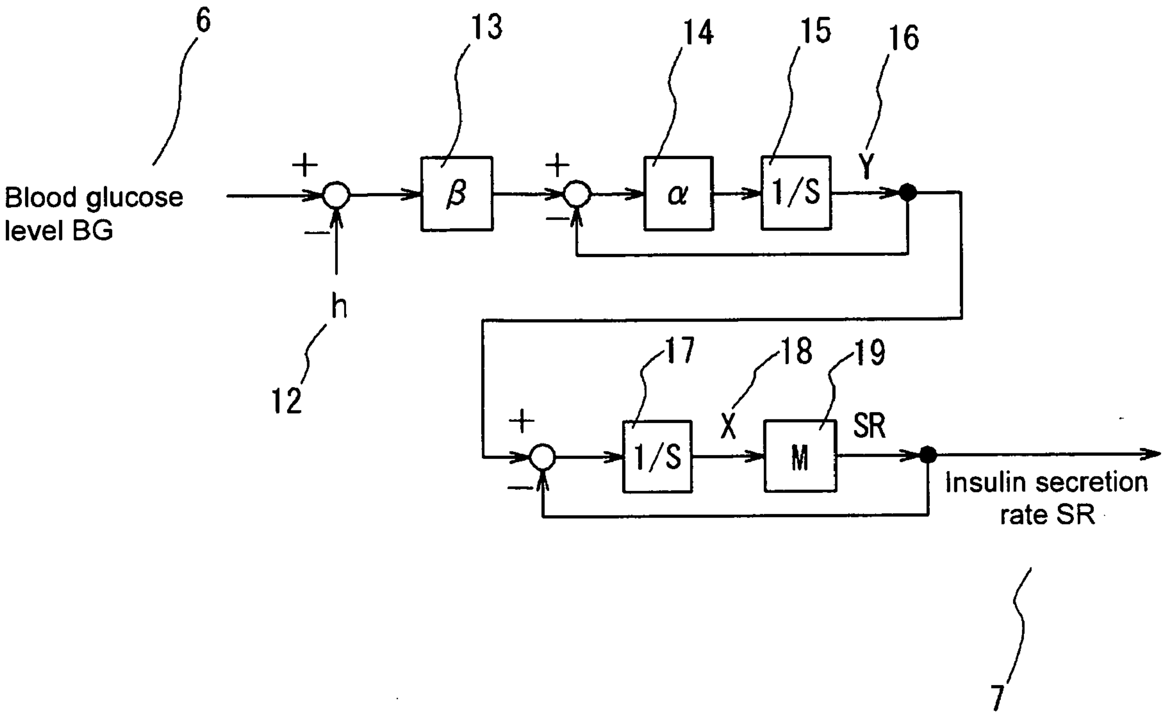
6
13
14
15
16
Blood glucose level BG
+
−
β
+
−
α
1/S
Y
h
12
17
18
19
+
−
1/S
X
M
SR
Insulin secretion rate SR
7

FIG. 6

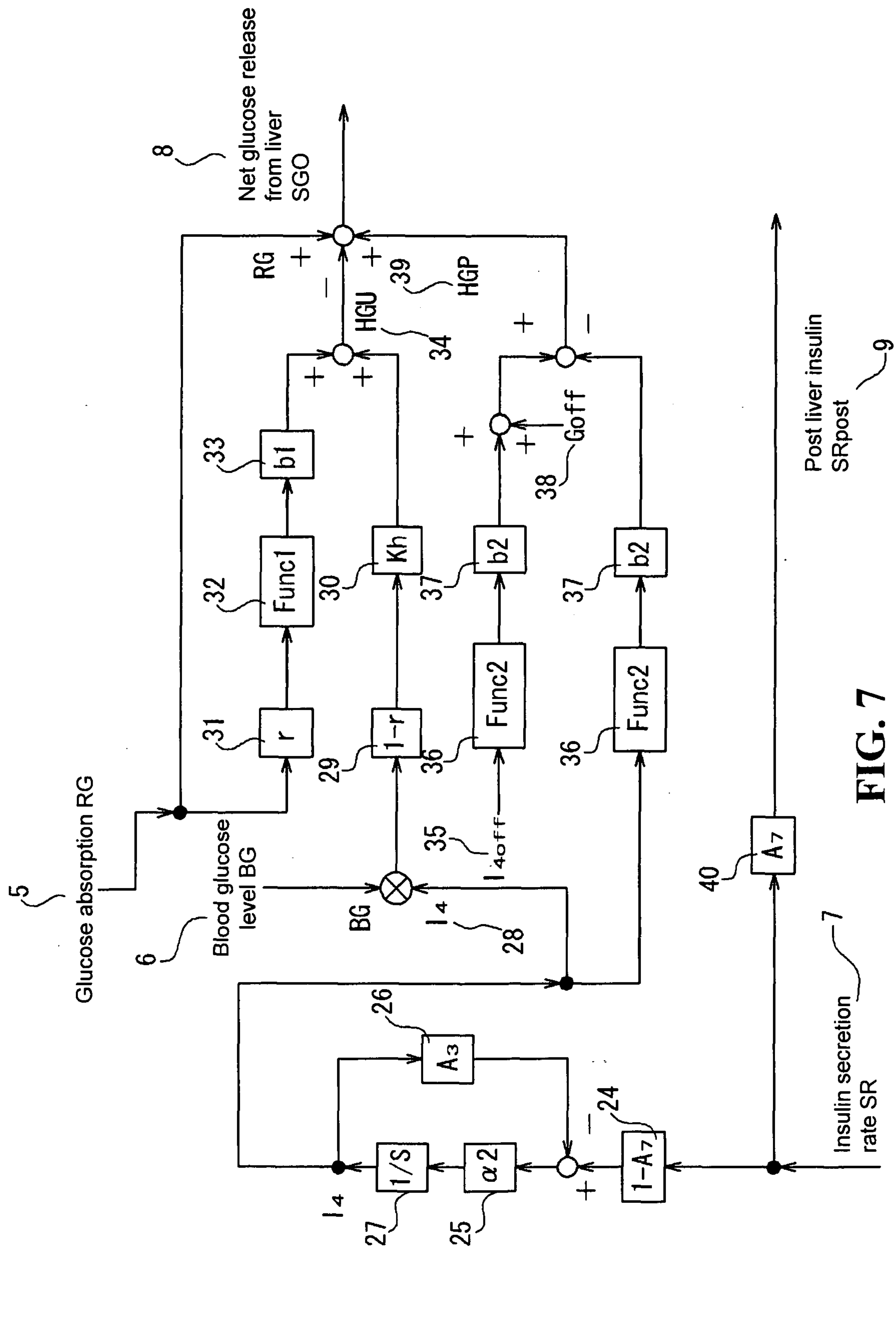
5
Glossary absorption RG
6
Blood glucose level BG
BG
8
Net glucose release from liver SGO
RG
HGU
34
39
HGP
Goff
38
Post liver insulin SRpost
9
Insulin secretion rate SR
7
31
r
32
Func1
33
b1
29
1-r
30
Kh
36
Func2
37
b2
35
$I_{4off}$
$I_4$
28
40
$A_7$
26
$A_3$
27
1/S
25
α2
24
$1-A_7$
$I_4$

FIG. 7

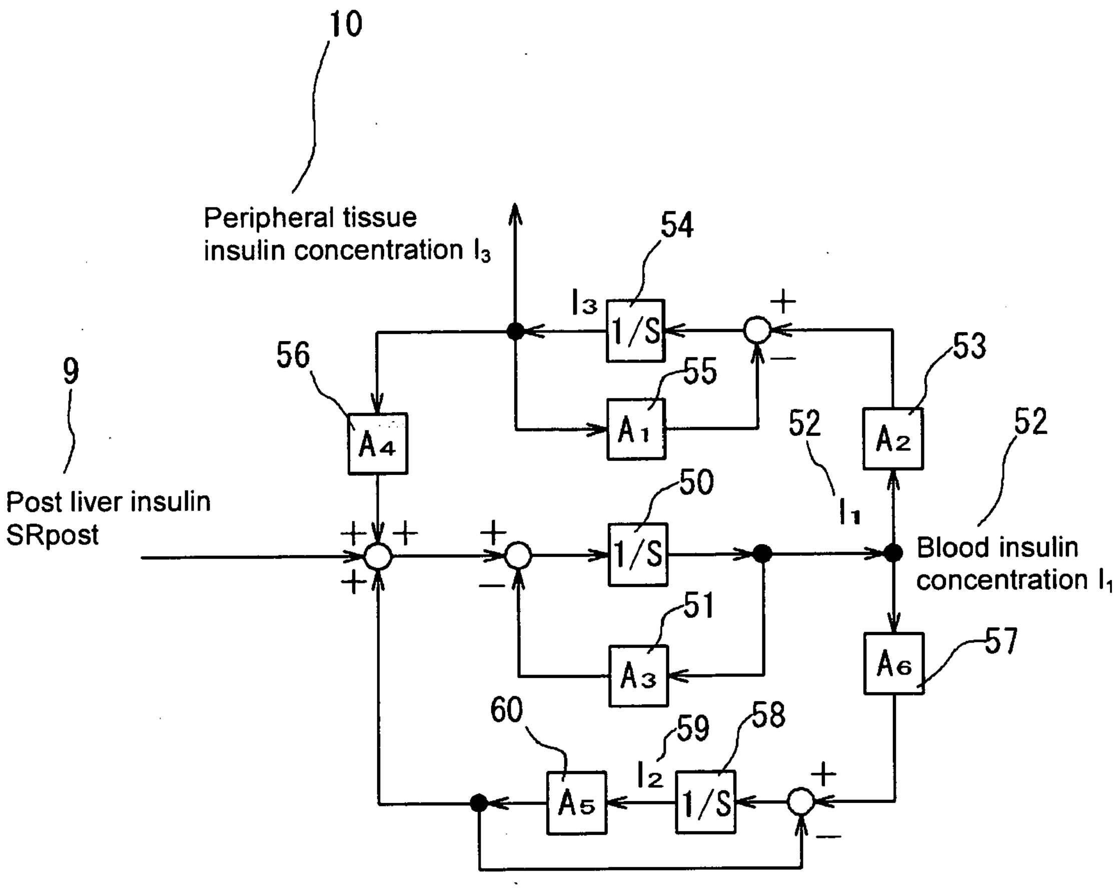
10
Peripheral tissue
insulin concentration $I_3$
54
$I_3$
1/S
+
−
55
$A_1$
53
$A_2$
56
$A_4$
9
Post liver insulin
SRpost
52
$I_1$
52
Blood insulin
concentration $I_1$
50
1/S
+
+
+
+
−
51
$A_3$
57
$A_6$
60
59
58
$I_2$
$A_5$
1/S
+
−

FIG. 8

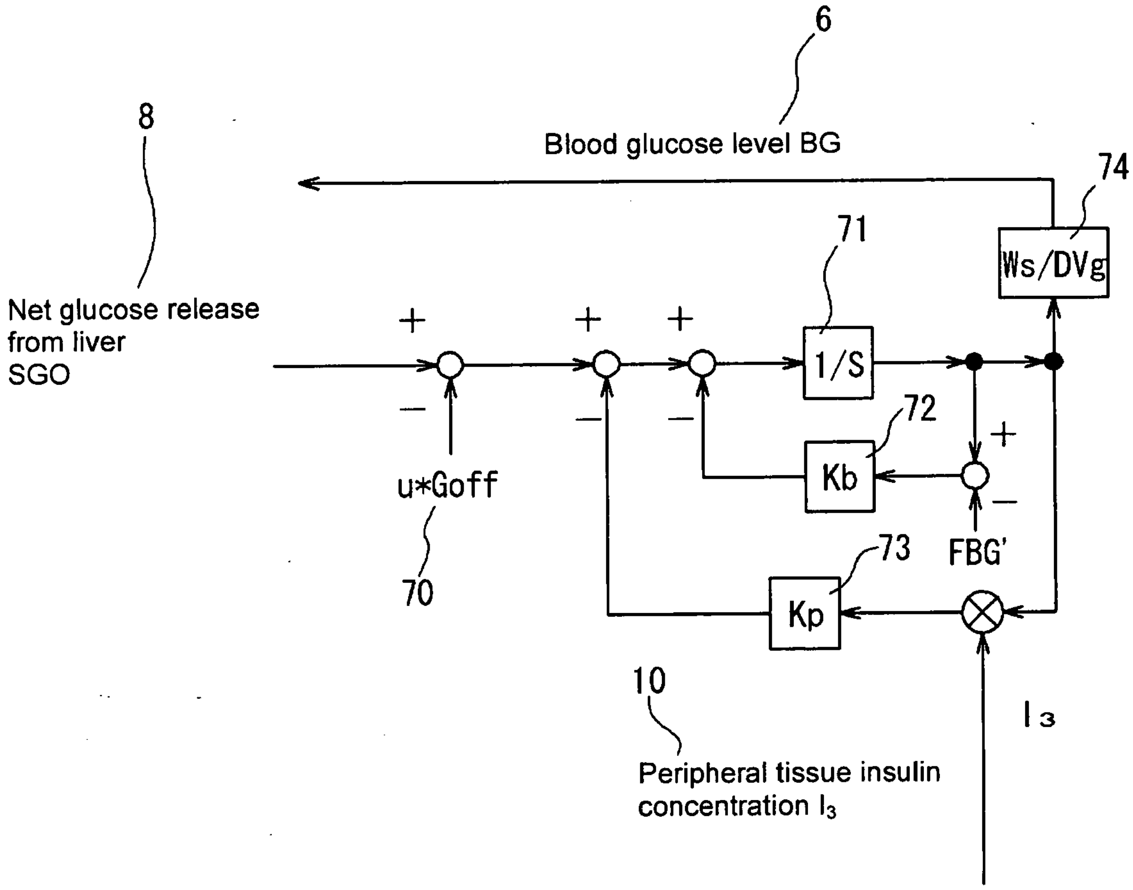
6
8
Blood glucose level BG
74
71
Ws/DVg
Net glucose release
from liver
SGO
+
+
+
1/S
−
−
−
72
+
u*Goff
Kb
−
73
FBG'
70
Kp
10
I3
Peripheral tissue insulin
concentration I3

FIG. 9

Template database DB1

| Template | Parameter set |
|---|---|
| T1 | PS#01 |
| T2 | PS#02 |
| T3 | PS#03 |
| ⋮ | ⋮ |
| ⋮ | ⋮ |

FIG. 10

(a)

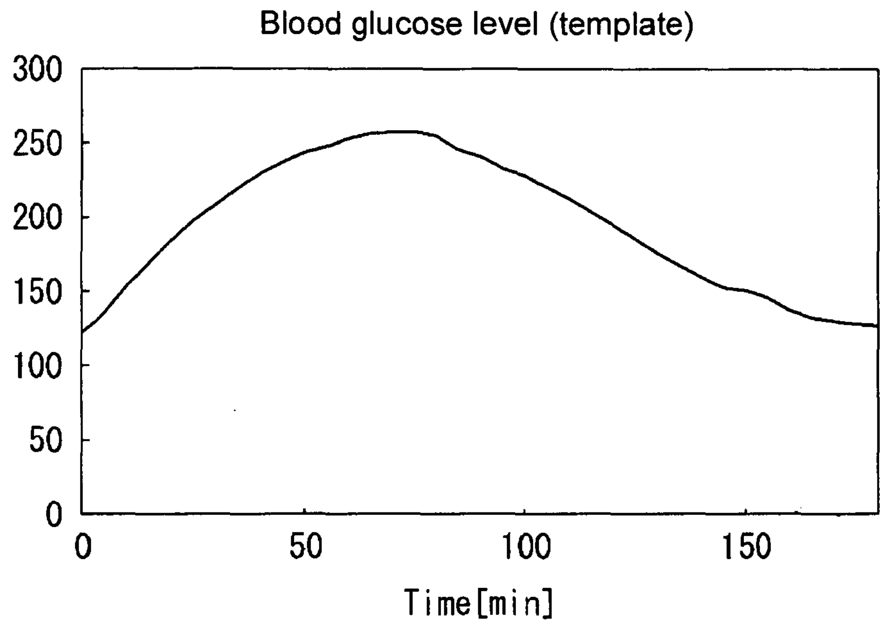
Blood glucose level (template)
300
250
200
150
100
50
0
0
50
100
150
Time[min]

(b)

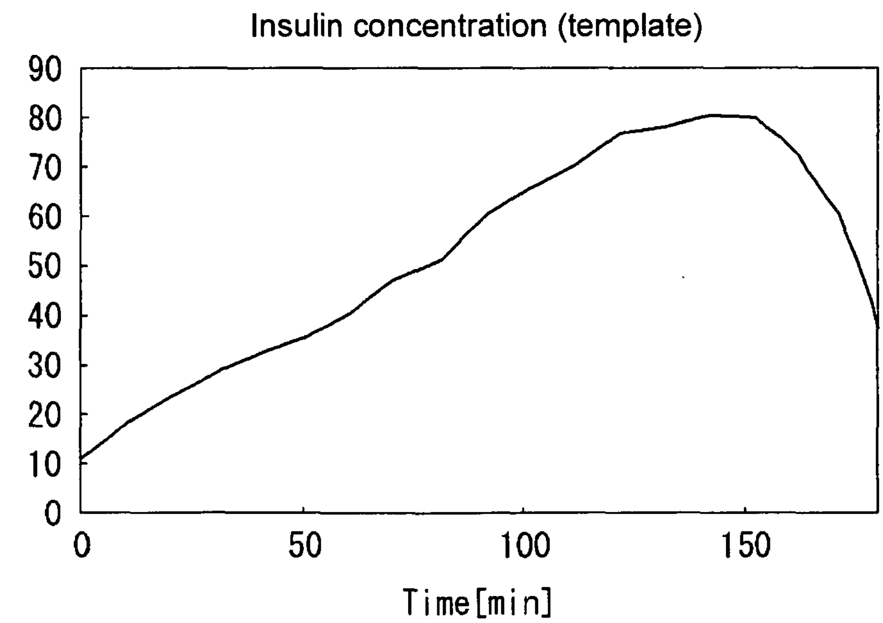
Insulin concentration (template)
90
80
70
60
50
40
30
20
10
0
0
50
100
150
Time[min]

FIG. 11

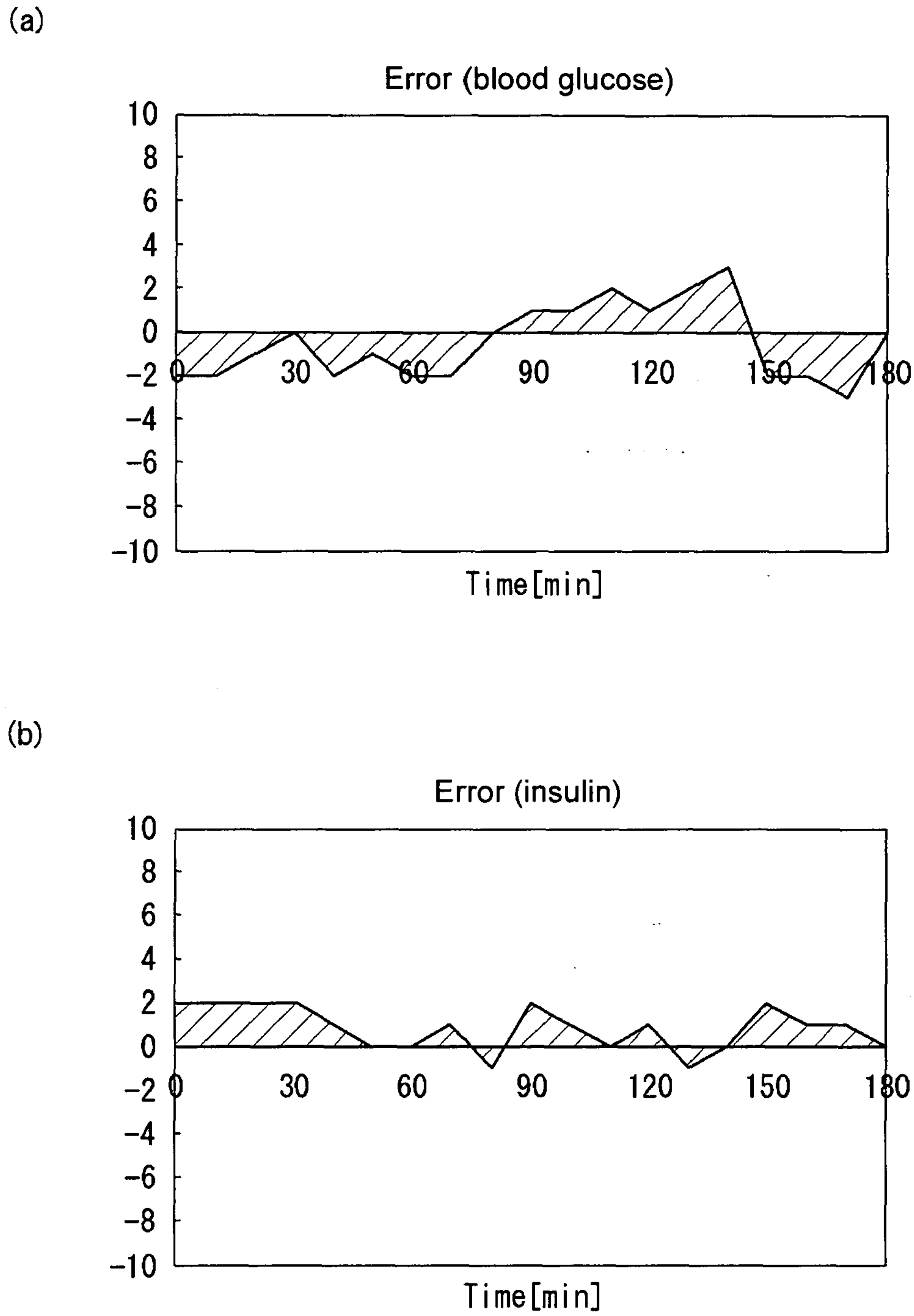
(a)
Error (blood glucose)
10
8
6
4
2
0
-2
-4
-6
-8
-10
0
30
60
90
120
150
180
Time[min]
(b)
Error (insulin)
10
8
6
4
2
0
-2
-4
-6
-8
-10
0
30
60
90
120
150
180
Time[min]

FIG. 12

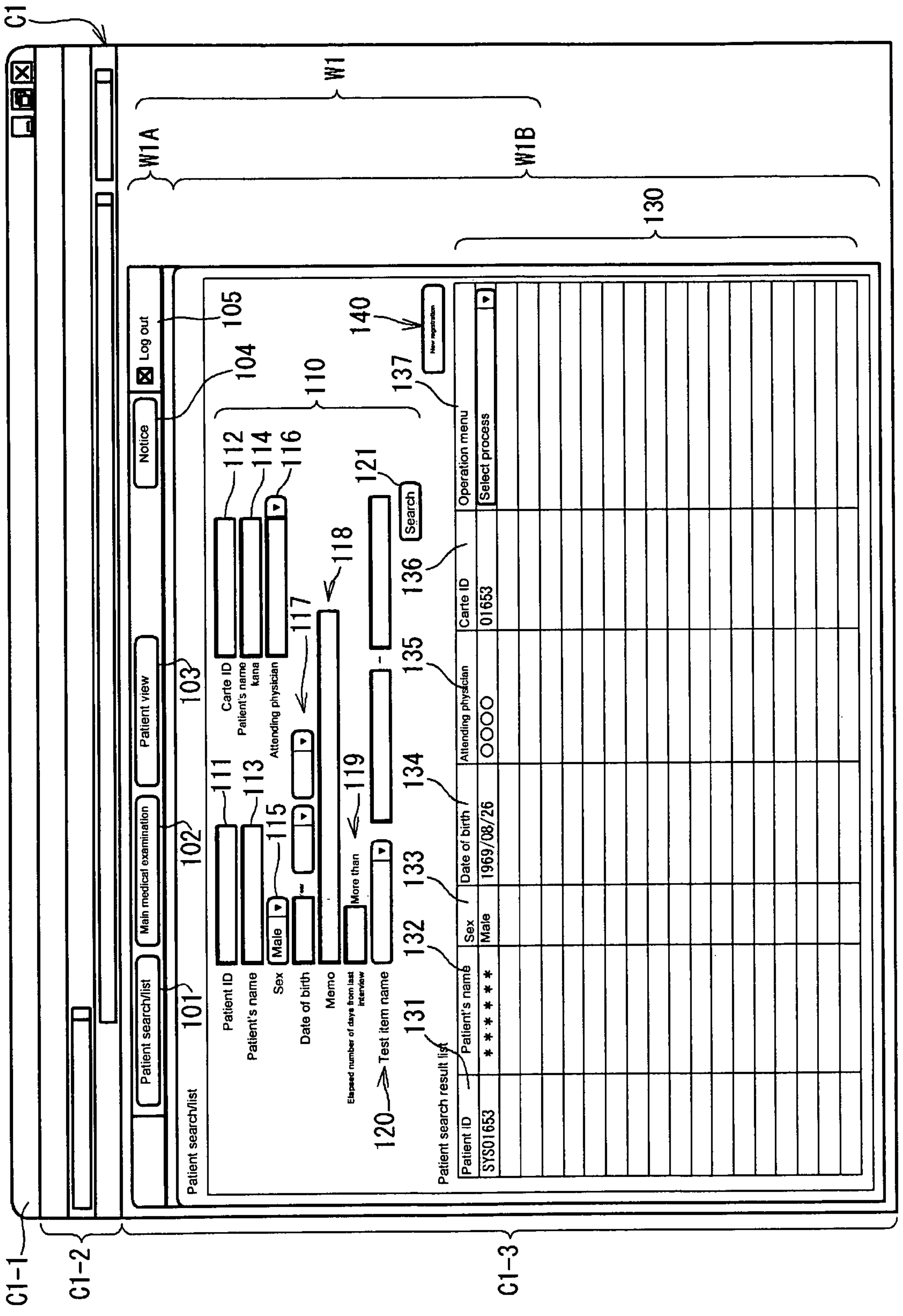

C1
C1-1
C1-2
C1-3
W1
W1A
W1B
Patient search/list
Main medical examination
Patient view
Notice
Log out
Patient search/list
Patient ID
Patient's name
Sex
Male
Date of birth
Memo
Elapsed number of days from last interview
More than
Test item name
Carte ID
Patient's name kana
Attending physician
Search
New registration
Patient search result list
Patient ID
Patient's name
Sex
Date of birth
Attending physician
Carte ID
Operation menu
SYS01653
* * * * * *
Male
1969/08/26
○○○○
01653
Select process
101
102
103
104
105
110
111
112
113
114
115
116
117
118
119
120
121
130
131
132
133
134
135
136
137
140


FIG. 13

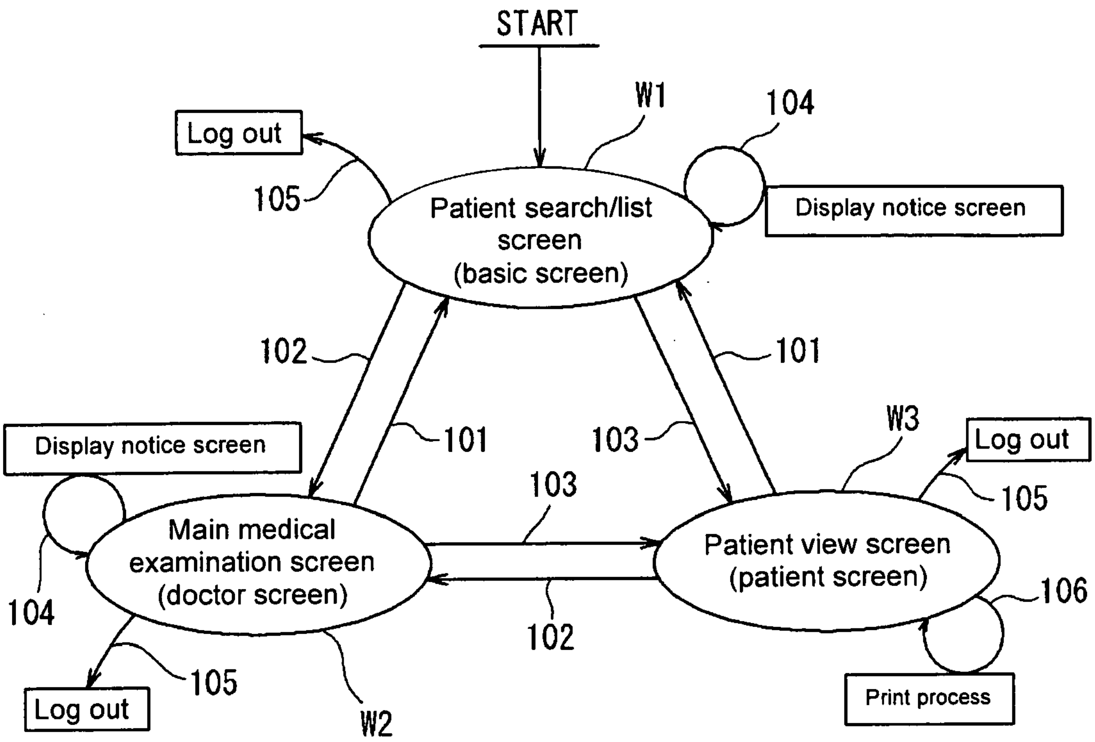
START
W1
104
Log out
105
Patient search/list screen (basic screen)
Display notice screen
102
101
Display notice screen
101
103
W3
Log out
103
105
Main medical examination screen (doctor screen)
Patient view screen (patient screen)
104
102
106
105
Log out
W2
Print process

FIG. 14

FIG.15

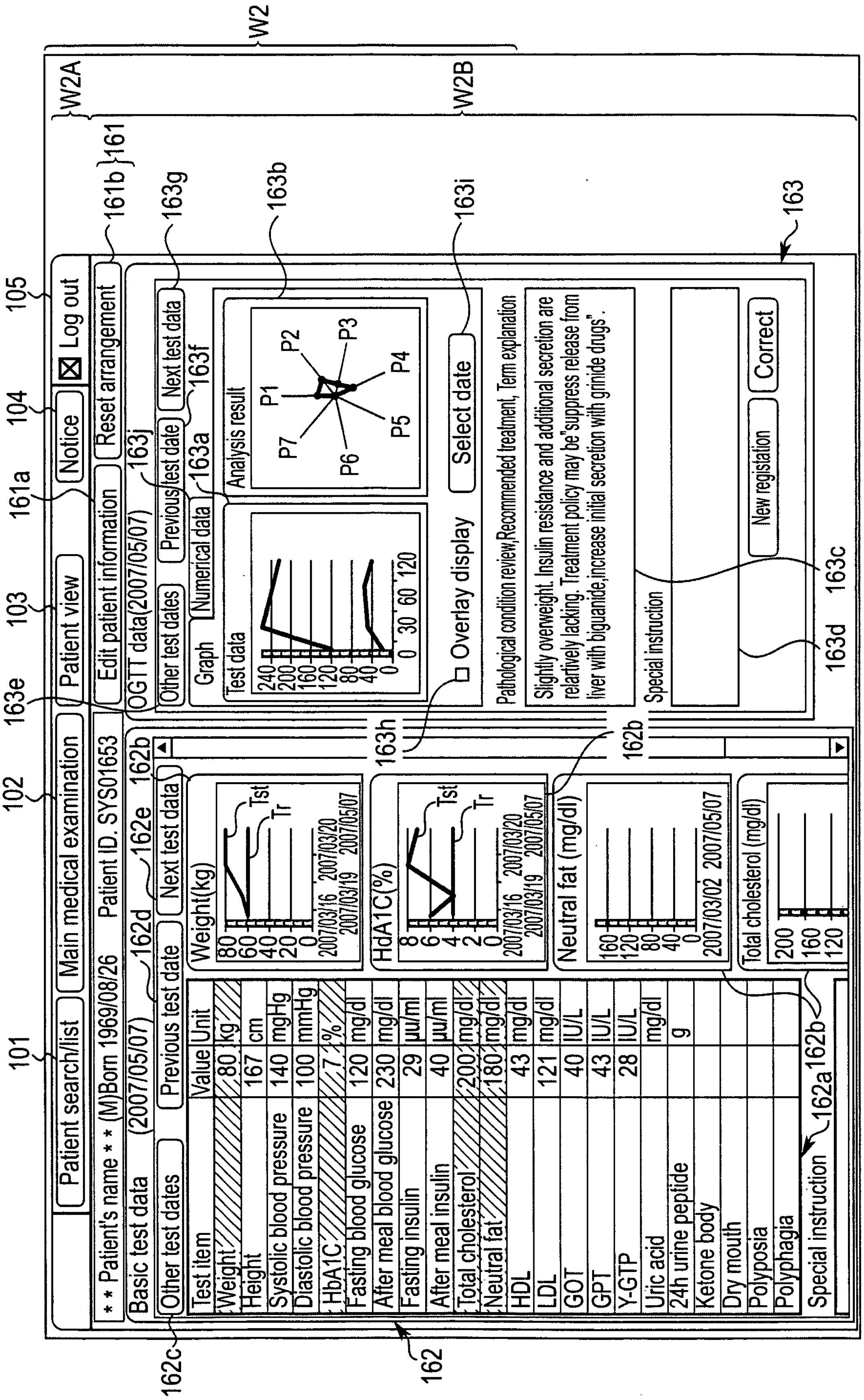
W2
W2A
W2B
101
102
103
104
105
161
161a
161b
162
162a
162b
162c
162d
162e
163
163a
163b
163c
163d
163e
163f
163g
163h
163i
163j
Patient search/list
Main medical examination
Patient view
Notice
Log out
* * Patient's name * * (M)Born 1969/08/26 Patient ID. SYS01653
Edit patient information
Reset arrangement
Basic test data (2007/05/07)
Other test dates
Previous test date
Next test data
Test item
Value
Unit
Weight 80 kg
Height 167 cm
Systolic blood pressure 140 mgHg
Diastolic blood pressure 100 mmHg
HbA1C 7 %
Fasting blood glucose 120 mg/dl
After meal blood glucose 230 mg/dl
Fasting insulin 29 μu/ml
After meal insulin 40 μu/ml
Total cholesterol 200 mg/dl
Neutral fat 180 mg/dl
HDL 43 mg/dl
LDL 121 mg/dl
GOT 40 IU/L
GPT 43 IU/L
Y-GTP 28 IU/L
Uric acid mg/dl
24h urine peptide g
Ketone body
Dry mouth
Polyposia
Polyphagia
Special instruction
Weight(kg)
HdA1C(%)
Neutral fat (mg/dl)
Total cholesterol (mg/dl)
Tst
Tr
2007/03/16
2007/03/19
2007/03/20
2007/05/07
2007/03/02
OGTT data(2007/05/07)
Graph
Numerical data
Test data
Analysis result
P1
P2
P3
P4
P5
P6
P7
Overlay display
Select date
Pathological condition review,Recommended treatment, Term explanation
Slightly overweight. Insulin resistance and additional secretion are relatively lacking. Treatment policy may be "suppress release from liver with biguanide,increase initial secretion with grinide drugs".
Special instruction
New registation
Correct

FIG.16

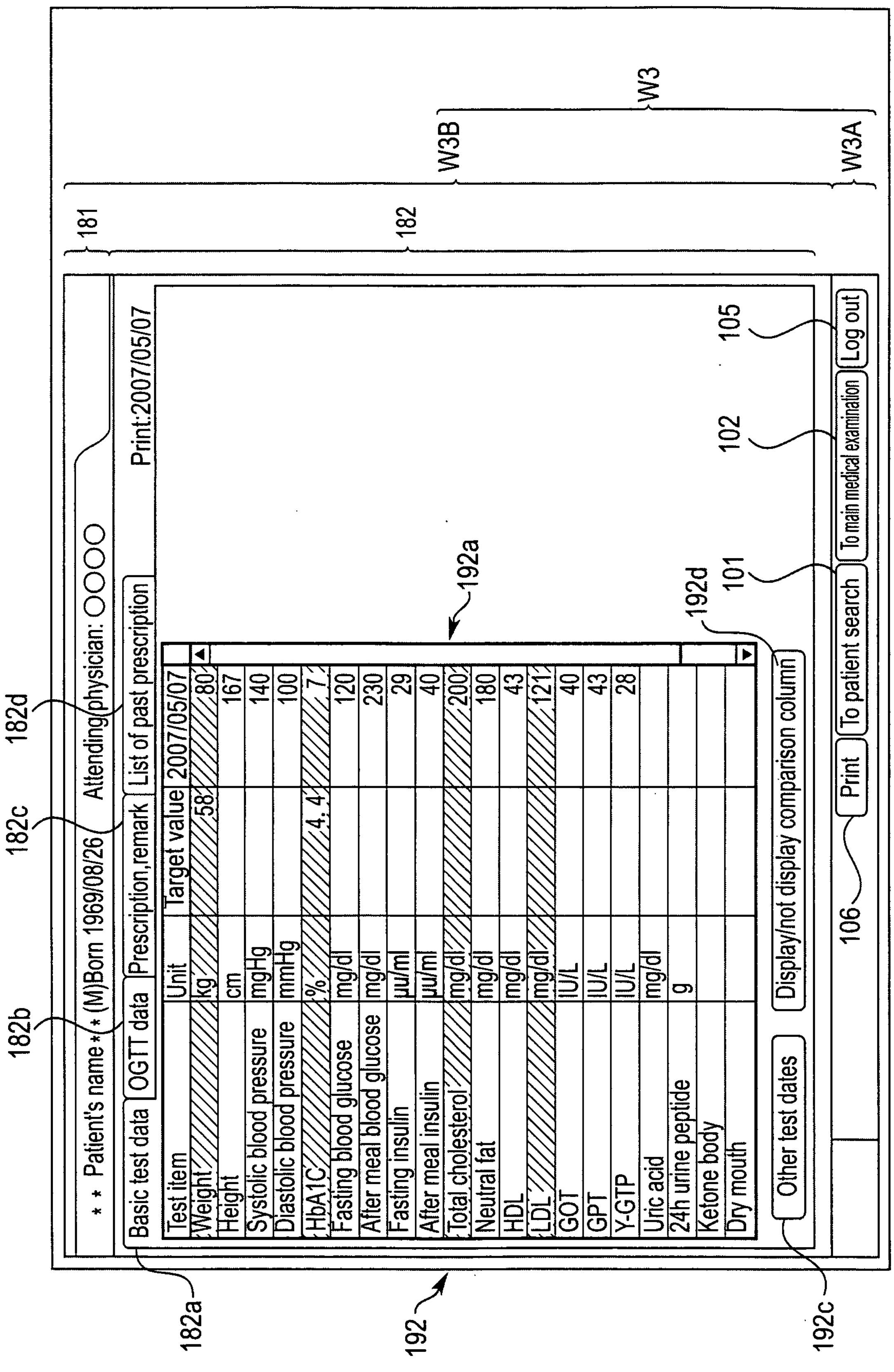

* * Patient's name * * (M)Born 1969/08/26 Attending physician: ○○○○
Basic test data
OGTT data
Prescription,remark
List of past prescription
Print:2007/05/07
Test item
Unit
Target value
2007/05/07
Weight kg 58 80
Height cm 167
Systolic blood pressure mgHg 140
Diastolic blood pressure mmHg 100
HbA1C % 4. 4 7
Fasting blood glucose mg/dl 120
After meal blood glucose mg/dl 230
Fasting insulin μu/ml 29
After meal insulin μu/ml 40
Total cholesterol mg/dl 200
Neutral fat mg/dl 180
HDL mg/dl 43
LDL mg/dl 121
GOT IU/L 40
GPT IU/L 43
Y-GTP IU/L 28
Uric acid mg/dl
24h urine peptide g
Ketone body
Dry mouth
Other test dates
Display/not display comparison column
Print
To patient search
To main medical examination
Log out
181
182
182a
182b
182c
182d
192
192a
192c
192d
101
102
105
106
W3
W3A
W3B

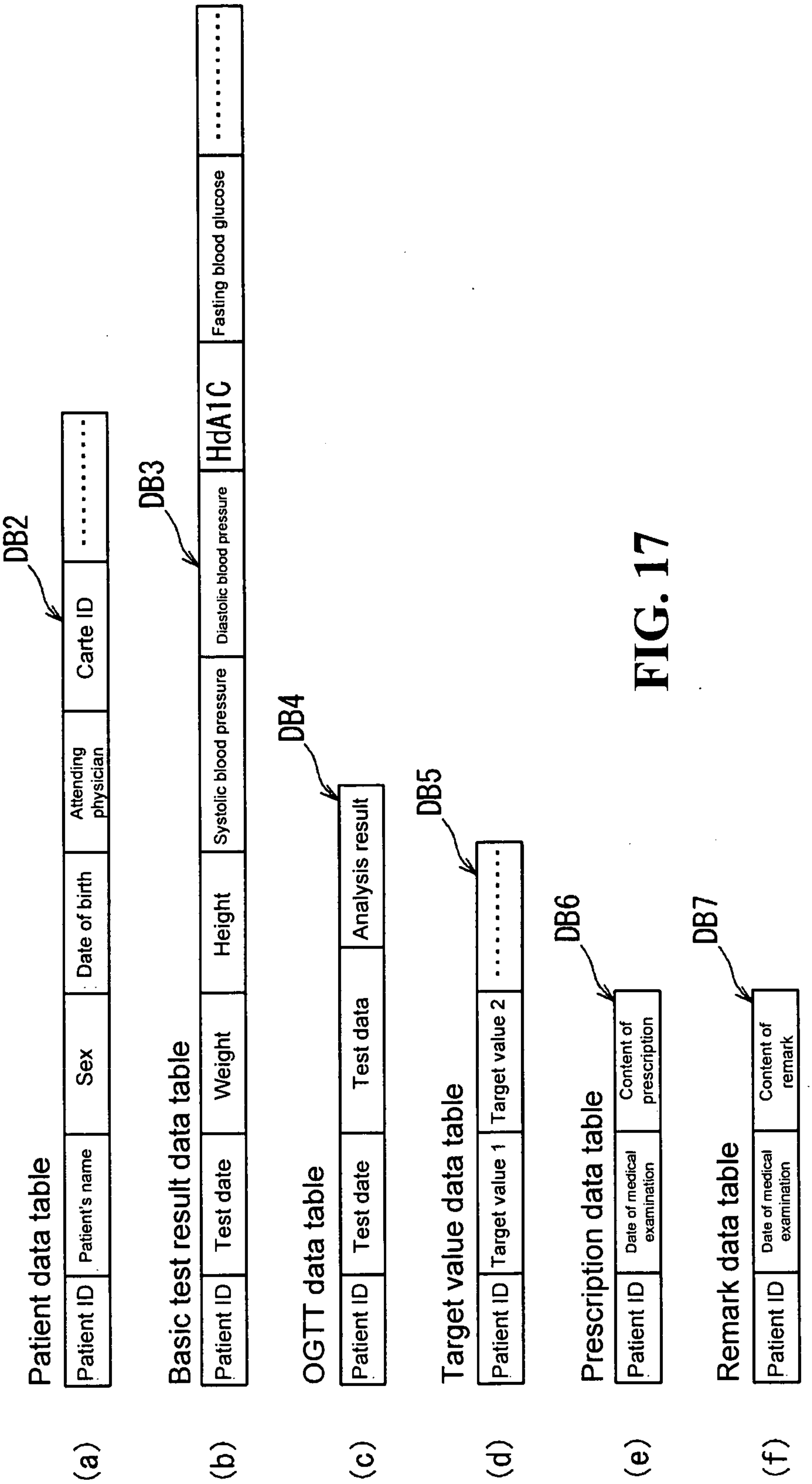
(a) Patient data table
Patient ID | Patient's name | Sex | Date of birth | Attending physician | Carte ID | ............
DB2
(b) Basic test result data table
Patient ID | Test date | Weight | Height | Systolic blood pressure | Diastolic blood pressure | HdA1C | Fasting blood glucose | ............
DB3
(c) OGTT data table
Patient ID | Test date | Test data | Analysis result
DB4
(d) Target value data table
Patient ID | Target value 1 | Target value 2 | ............
DB5
(e) Prescription data table
Patient ID | Date of medical examination | Content of prescription
DB6
(f) Remark data table
Patient ID | Date of medical examination | Content of remark
DB7

FIG. 17

FIG.18

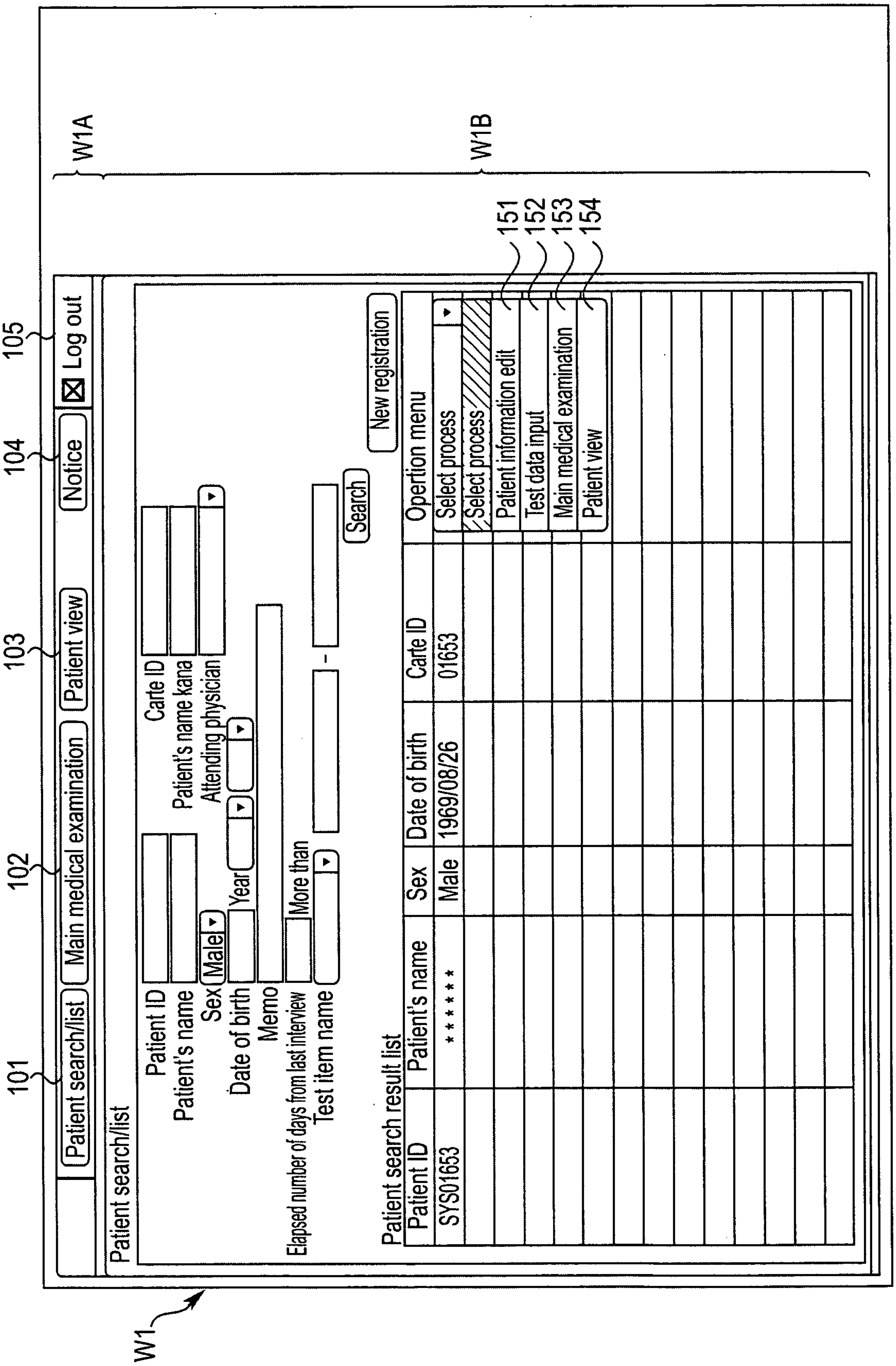
101
102
103
104
105
Patient search/list
Main medical examination
Patient view
Notice
Log out
W1A
W1B
Patient search/list
Patient ID
Patient's name
Sex
Male
Date of birth
Year
Memo
Elapsed number of days from last interview
More than
Test item name
Carte ID
Patient's name kana
Attending physician
Search
New registration
Patient search result list
Patient ID
Patient's name
Sex
Date of birth
Carte ID
Opertion menu
SYS01653
******
Male
1969/08/26
01653
Select process
Select process
Patient information edit
Test data input
Main medical examination
Patient view
151
152
153
154
W1

FIG.19

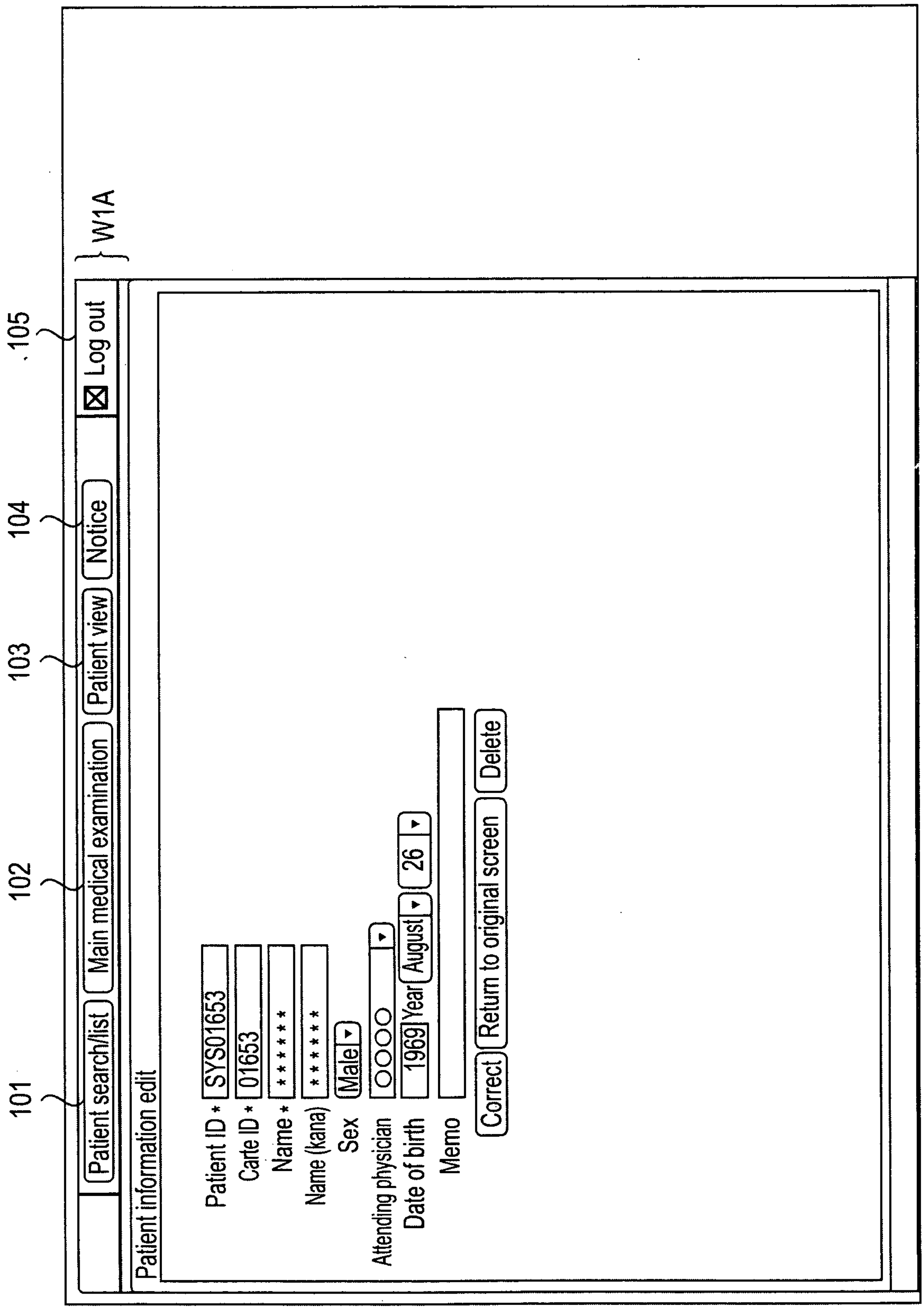
101
102
103
104
105
W1A
Patient search/list
Main medical examination
Patient view
Notice
Log out
Patient information edit
Patient ID *
SYS01653
Carte ID *
01653
Name *
******
Name (kana)
******
Sex
Male
Attending physician
○○○○
Date of birth
1969
Year
August
26
Memo
Correct
Return to original screen
Delete

FIG.20

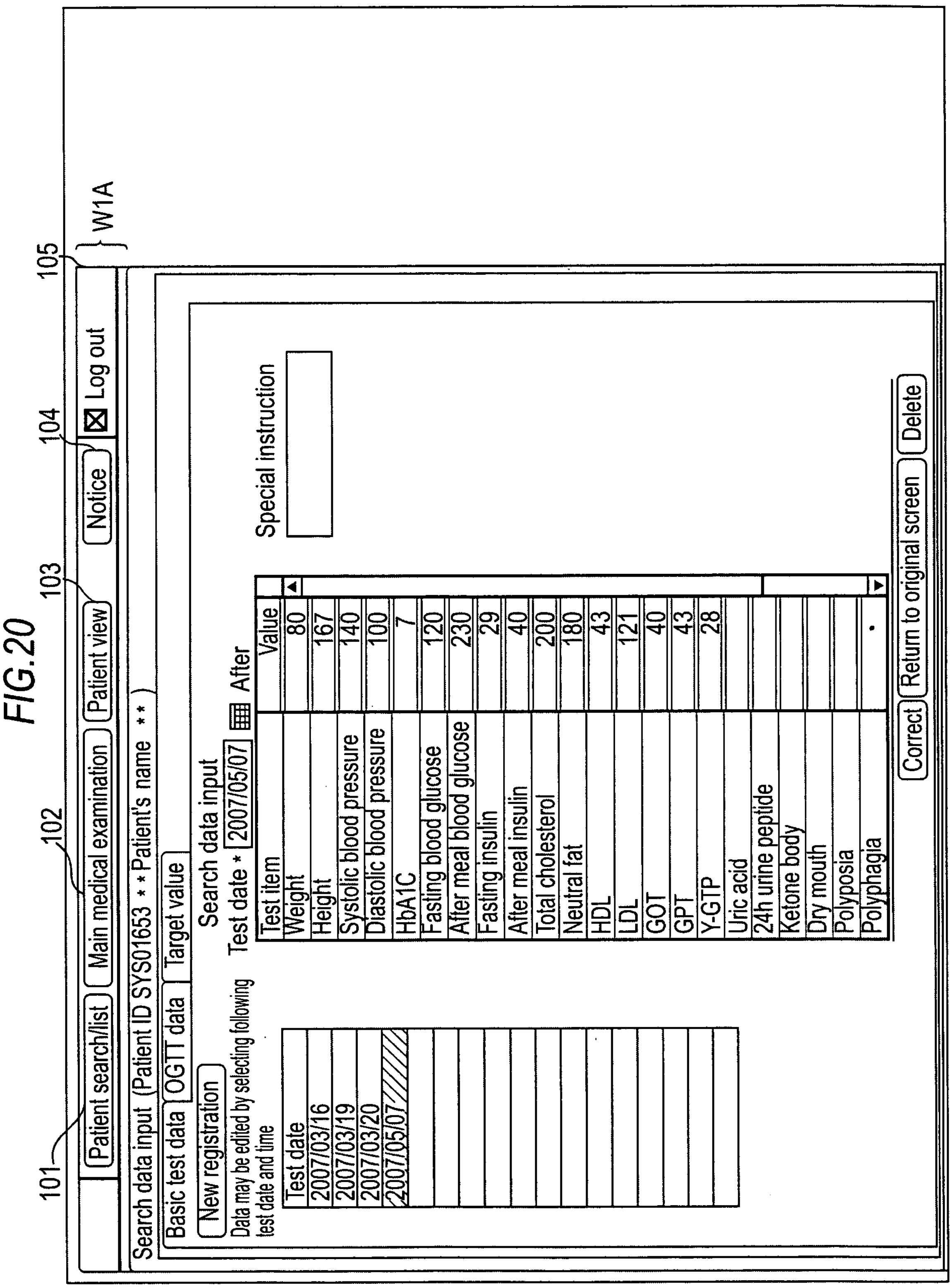
101
102
103
104
105
W1A
Patient search/list
Main medical examination
Patient view
Notice
Log out
Search data input (Patient ID SYS01653 * * Patient's name * *)
Basic test data
OGTT data
Target value
New registration
Data may be edited by selecting following test date and time
Test date
2007/03/16
2007/03/19
2007/03/20
2007/05/07
Search data input
Test date * 2007/05/07
After
Test item
Value
Weight 80
Height 167
Systolic blood pressure 140
Diastolic blood pressure 100
HbA1C 7
Fasting blood glucose 120
After meal blood glucose 230
Fasting insulin 29
After meal insulin 40
Total cholesterol 200
Neutral fat 180
HDL 43
LDL 121
GOT 40
GPT 43
Y-GTP 28
Uric acid
24h urine peptide
Ketone body
Dry mouth
Polyposia
Polyphagia
Special instruction
Correct
Return to original screen
Delete

*FIG.21*

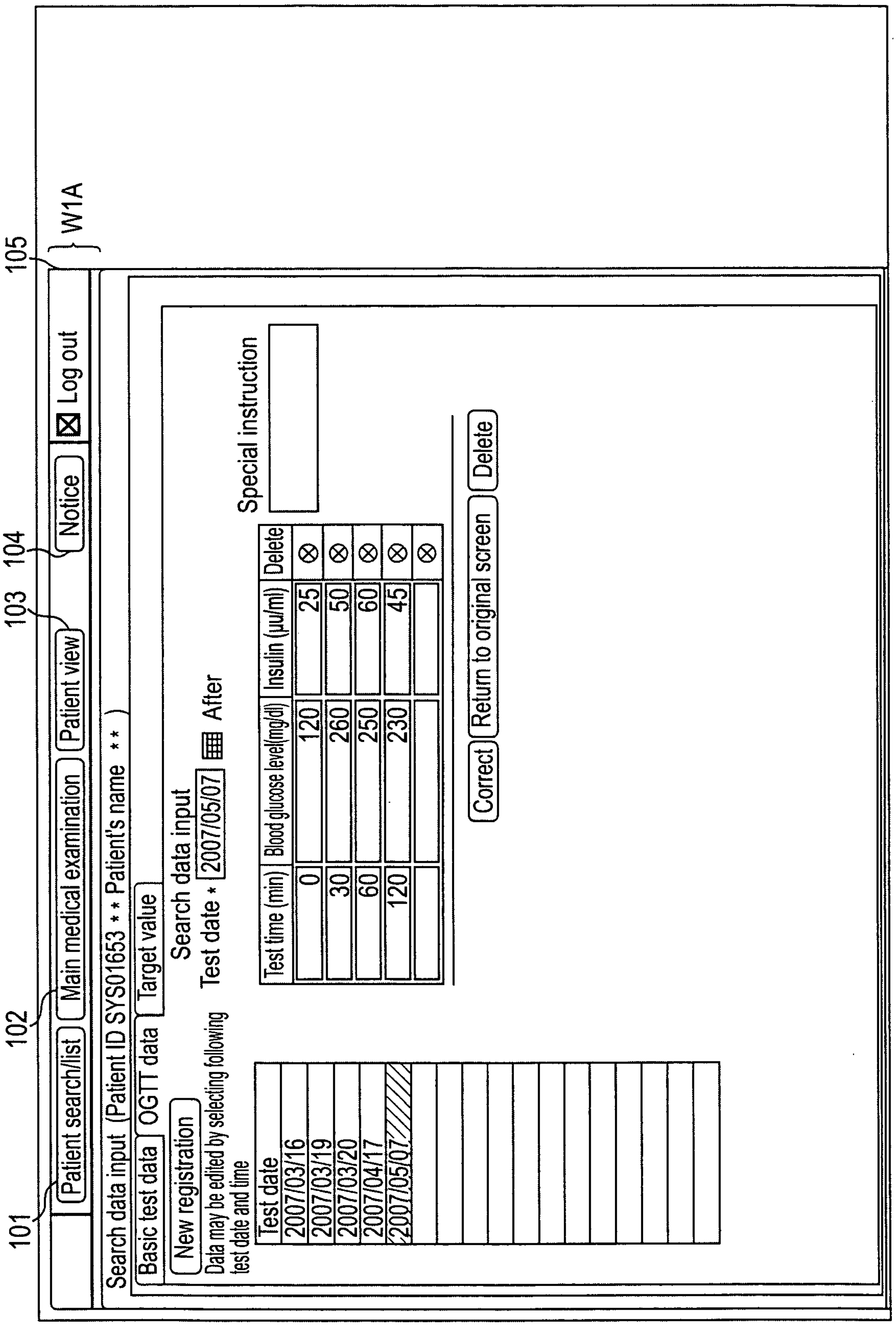
101
102
103
104
105
W1A
Patient search/list
Main medical examination
Patient view
Notice
Log out
Search data input (Patient ID SYS01653 * * Patient's name * * )
Basic test data
OGTT data
Target value
New registration
Data may be edited by selecting following test date and time
Test date
2007/03/16
2007/03/19
2007/03/20
2007/04/17
2007/05/07
Search data input
Test date * 2007/05/07 After
Test time (min)
Blood glucose level(mg/dl)
Insulin (μu/ml)
Delete
0
120
25
30
260
50
60
250
60
120
230
45
Special instruction
Correct
Return to original screen
Delete

FIG.22

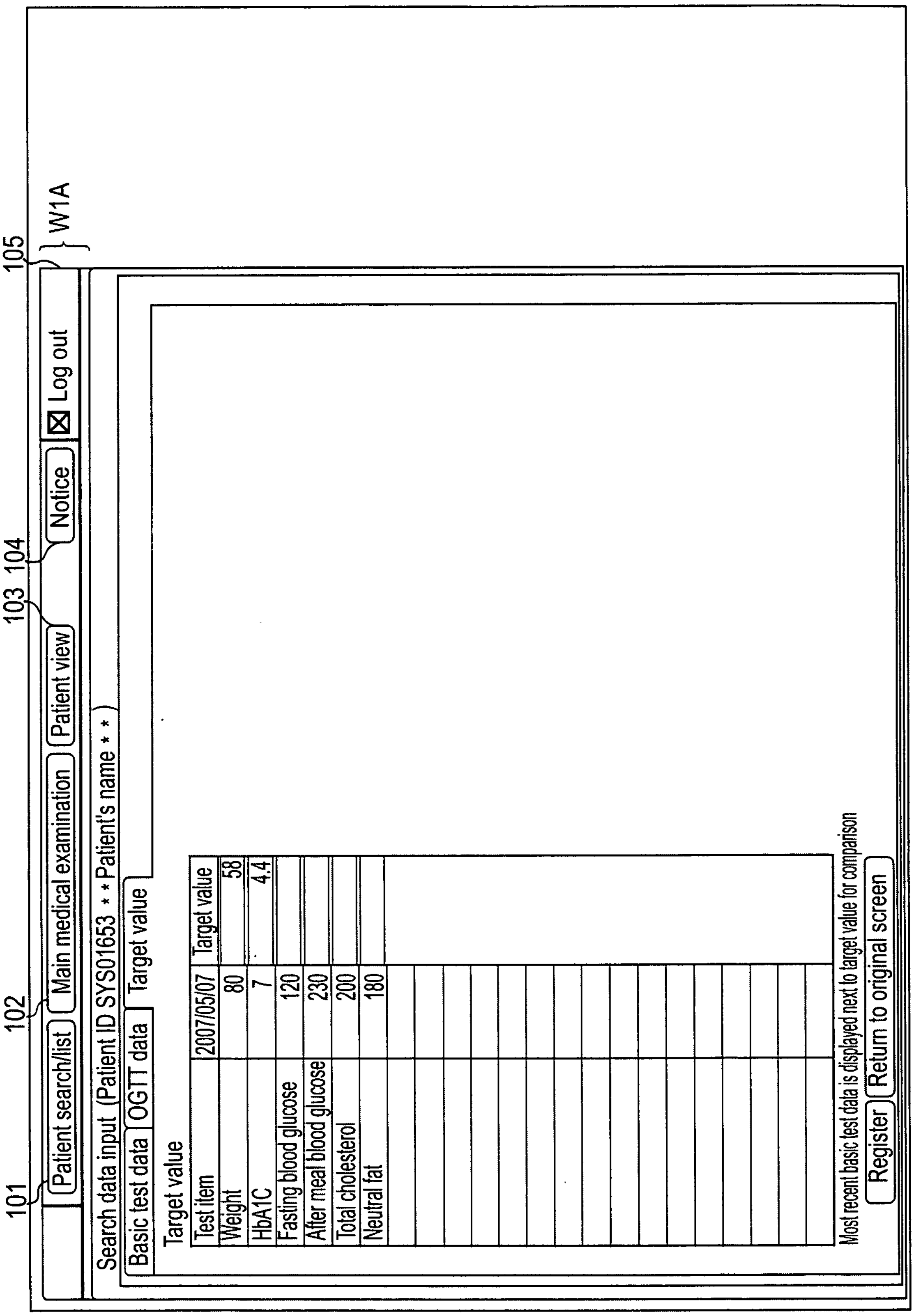
101
102
103
104
105
W1A
Patient search/list
Main medical examination
Patient view
Notice
Log out
Search data input (Patient ID SYS01653 * * Patient's name * * )
Basic test data
OGTT data
Target value
Target value
Test item
2007/05/07
Target value
Weight
80
58
HbA1C
7
4.4
Fasting blood glucose
120
After meal blood glucose
230
Total cholesterol
200
Neutral fat
180
Most recent basic test data is displayed next to target value for comparison
Register
Return to original screen

FIG.23

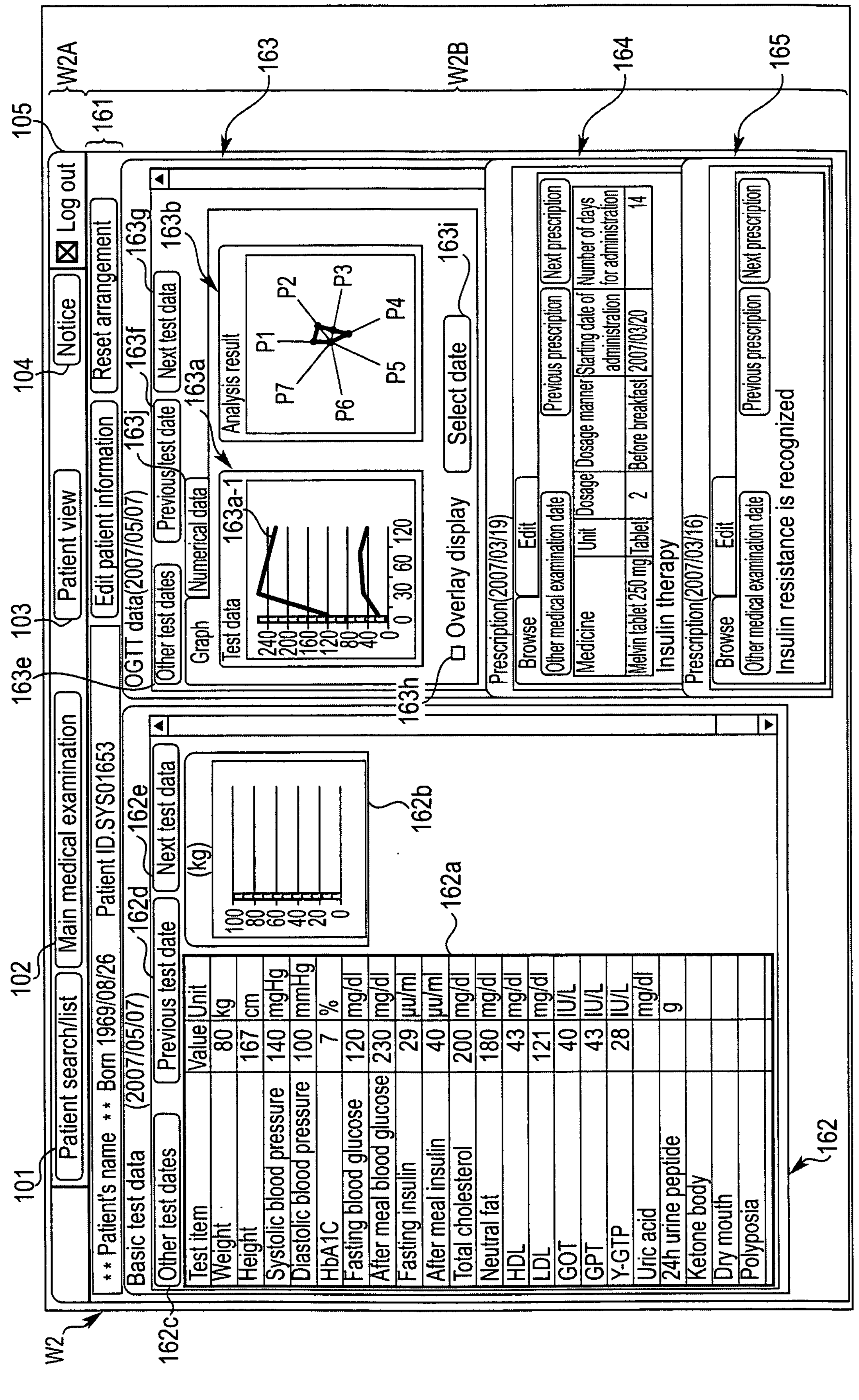
101
102
103
104
105
W2A
W2B
161
162
163
164
165
W2
Patient search/list
Main medical examination
Patient view
Notice
Log out
** Patient's name ** Born 1969/08/26 Patient ID.SYS01653
Edit patient information
Reset arrangement
Basic test data (2007/05/07)
Other test dates
Previous test date
Next test data
162a
162b
162c
162d
162e
Test item
Value
Unit
Weight 80 kg
Height 167 cm
Systolic blood pressure 140 mgHg
Diastolic blood pressure 100 mmHg
HbA1C 7 %
Fasting blood glucose 120 mg/dl
After meal blood glucose 230 mg/dl
Fasting insulin 29 μu/ml
After meal insulin 40 μu/ml
Total cholesterol 200 mg/dl
Neutral fat 180 mg/dl
HDL 43 mg/dl
LDL 121 mg/dl
GOT 40 IU/L
GPT 43 IU/L
Y-GTP 28 IU/L
Uric acid mg/dl
24h urine peptide g
Ketone body
Dry mouth
Polyposia
(kg)
100
80
60
40
20
0
OGTT data(2007/05/07)
Other test dates
Previous test date
Next test data
163a
163a-1
163b
163e
163f
163g
163h
163i
163j
Graph
Numerical data
Test data
240
200
160
120
80
40
0
0 30 60 120
Analysis result
P1
P2
P3
P4
P5
P6
P7
Overlay display
Select date
Prescription(2007/03/19)
Browse
Edit
Other medical examination date
Previous prescription
Next prescription
Medicine
Unit
Dosage
Dosage manner
Starting date of administration
Number of days for administration
Melvin tablet 250 mg Tablet 2 Before breakfast 2007/03/20 14
Insulin therapy
Prescription(2007/03/16)
Browse
Edit
Other medical examination date
Previous prescription
Next prescription
Insulin resistance is recognized

*FIG.24*

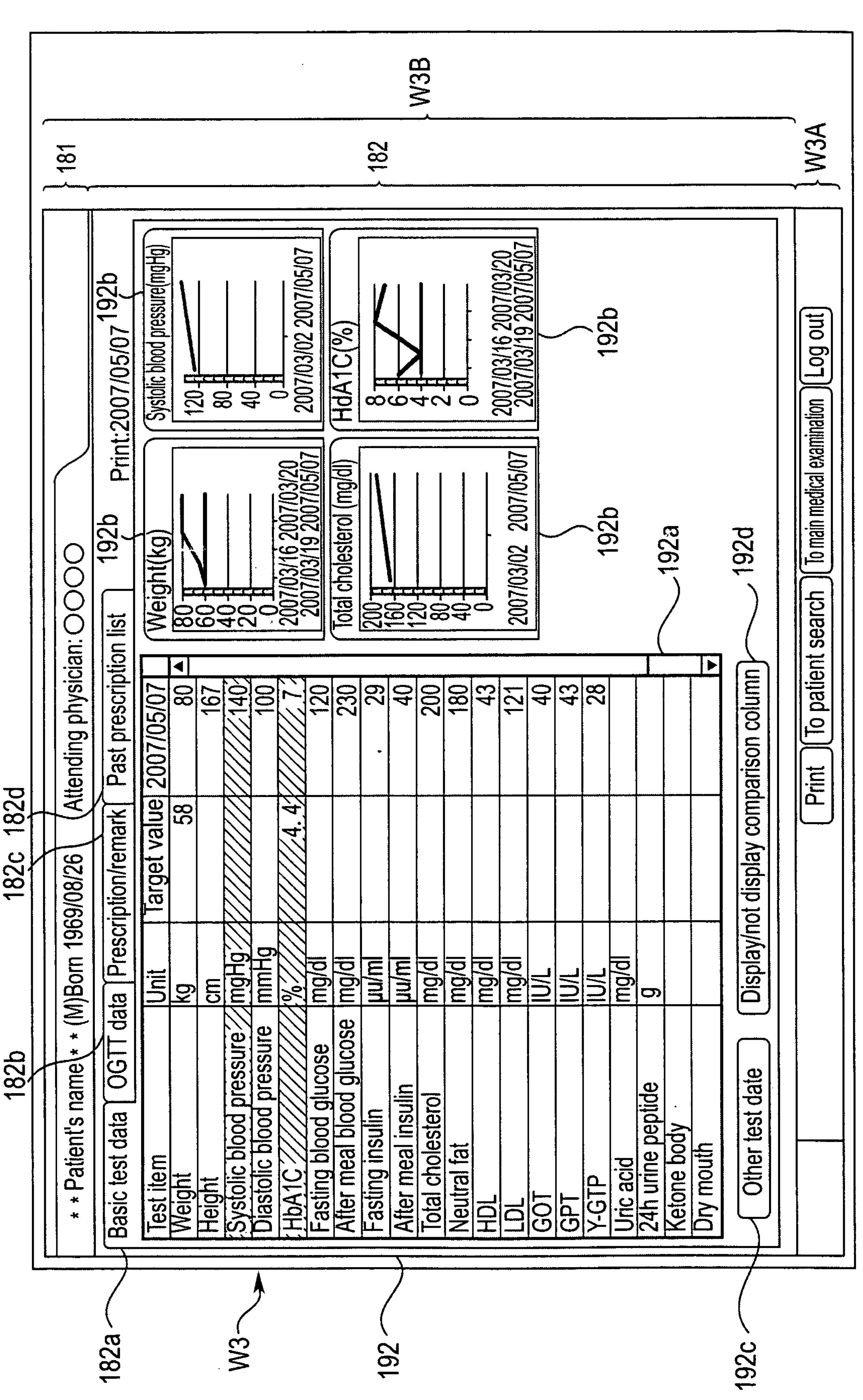

W3B
181
182
W3A
* * Patient's name* * (M)Born 1969/08/26
Attending physician: ○○○○
Basic test data
OGTT data
Prescription/remark
Past prescription list
Print:2007/05/07
Test item
Unit
Target value
2007/05/07
Weight
kg
58
80
Height
cm
167
Systolic blood pressure
mmHg
140
Diastolic blood pressure
mmHg
100
HbA1C
%
4. 4
7
Fasting blood glucose
mg/dl
120
After meal blood glucose
mg/dl
230
Fasting insulin
μu/ml
29
After meal insulin
μu/ml
40
Total cholesterol
mg/dl
200
Neutral fat
mg/dl
180
HDL
mg/dl
43
LDL
mg/dl
121
GOT
IU/L
40
GPT
IU/L
43
Y-GTP
IU/L
28
Uric acid
mg/dl
24h urine peptide
g
Ketone body
Dry mouth
Other test date
Display/not display comparison column
Weight(kg)
2007/03/16 2007/03/20
2007/03/19 2007/05/07
Systolic blood pressure(mmHg)
2007/03/02 2007/05/07
Total cholesterol (mg/dl)
2007/03/02 2007/05/07
HdA1C(%)
2007/03/16 2007/03/20
2007/03/19 2007/05/07
Print
To patient search
To main medical examination
Log out
182a
182b
182c
182d
W3
192
192a
192b
192c
192d

FIG.25

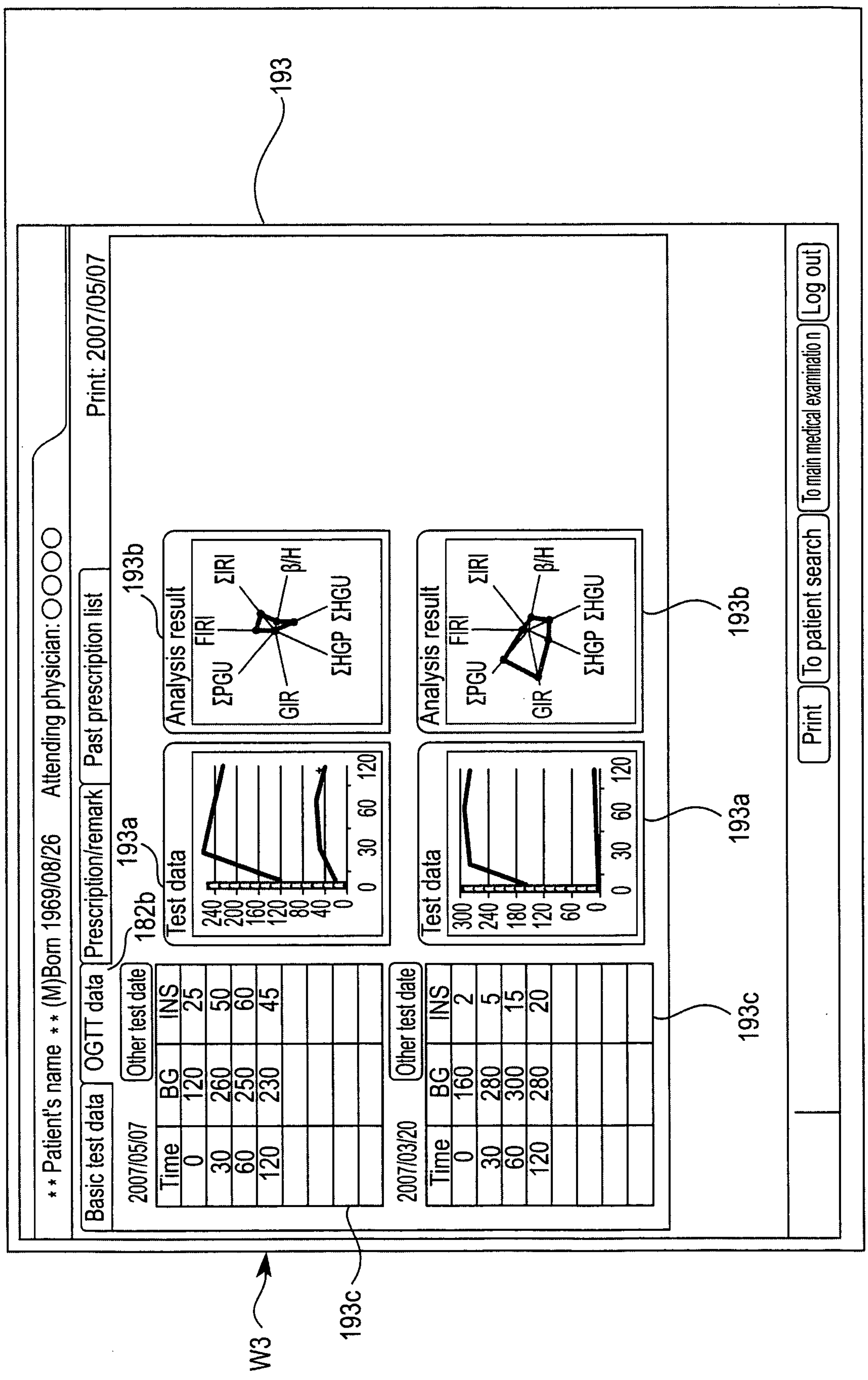

193
* * Patient's name * * (M)Born 1969/08/26 Attending physician:○○○○
Basic test data
OGTT data
Prescription/remark
Past prescription list
Print: 2007/05/07
182b
Other test date
2007/05/07
Time BG INS
0 120 25
30 260 50
60 250 60
120 230 45
2007/03/20
Time BG INS
0 160 2
30 280 5
60 300 15
120 280 20
193a
Test data
193b
Analysis result
FIRI
ΣIRI
β/H
ΣHGU
ΣHGP
GIR
ΣPGU
193c
Print
To patient search
To main medical examination
Log out
W3

FIG.26

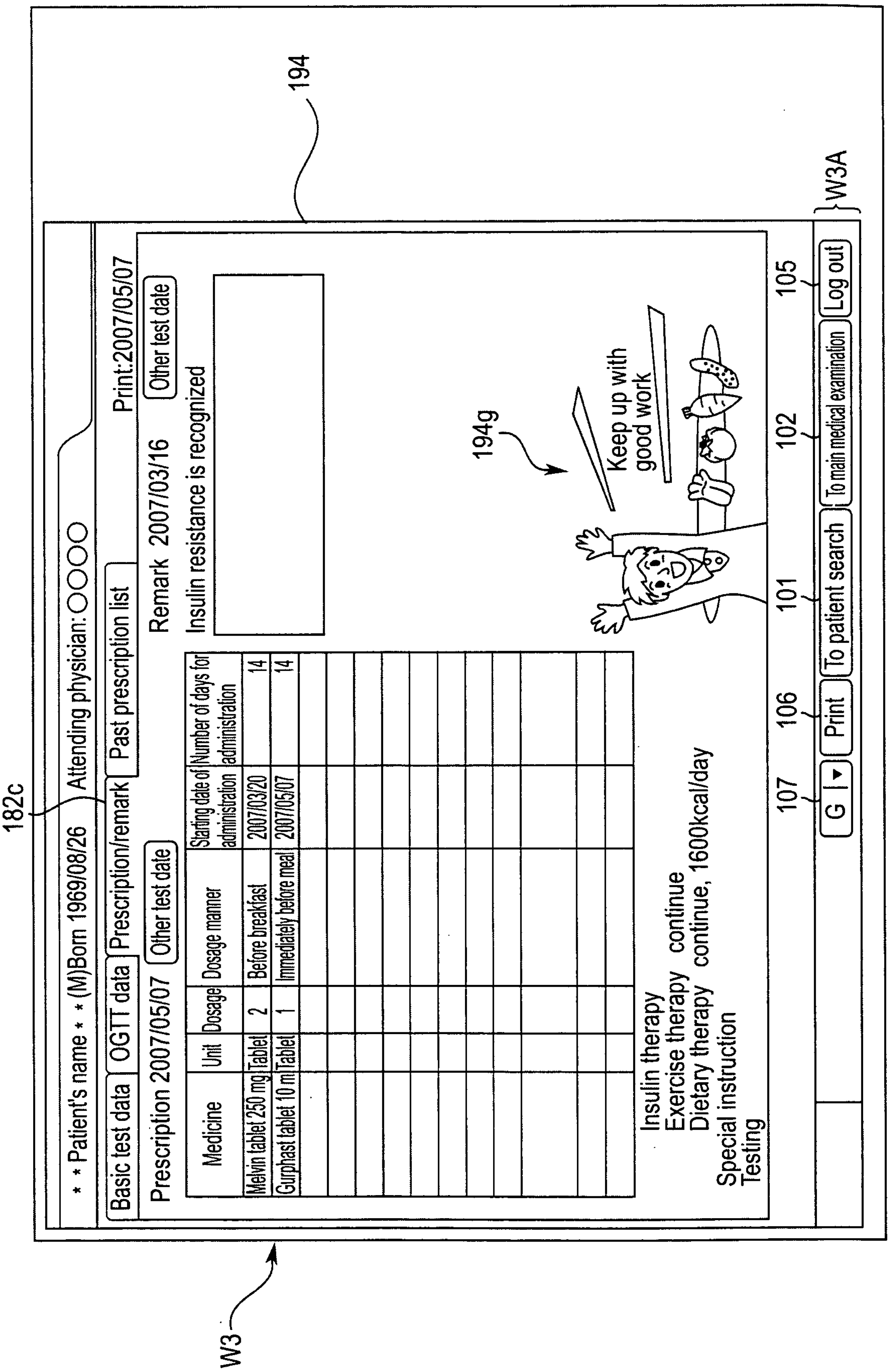

182c
* * Patient's name * * (M)Born 1969/08/26 Attending physician:○○○○
Basic test data
OGTT data
Prescription/remark
Past prescription list
Prescription 2007/05/07
Other test date
Medicine
Unit
Dosage
Dosage manner
Starting date of administration
Number of days for administration
Melvin tablet 250 mg Tablet 2 Before breakfast 2007/03/20 14
Gurphast tablet 10 ml Tablet 1 Immediately before meal 2007/05/07 14
Insulin therapy
Exercise therapy continue
Dietary therapy continue, 1600kcal/day
Special instruction
Testing
Remark 2007/03/16
Print:2007/05/07
Other test date
Insulin resistance is recognized
194g
Keep up with good work
194
G
Print
To patient search
To main medical examination
Log out
107
106
101
102
105
W3A
W3

*FIG.27*

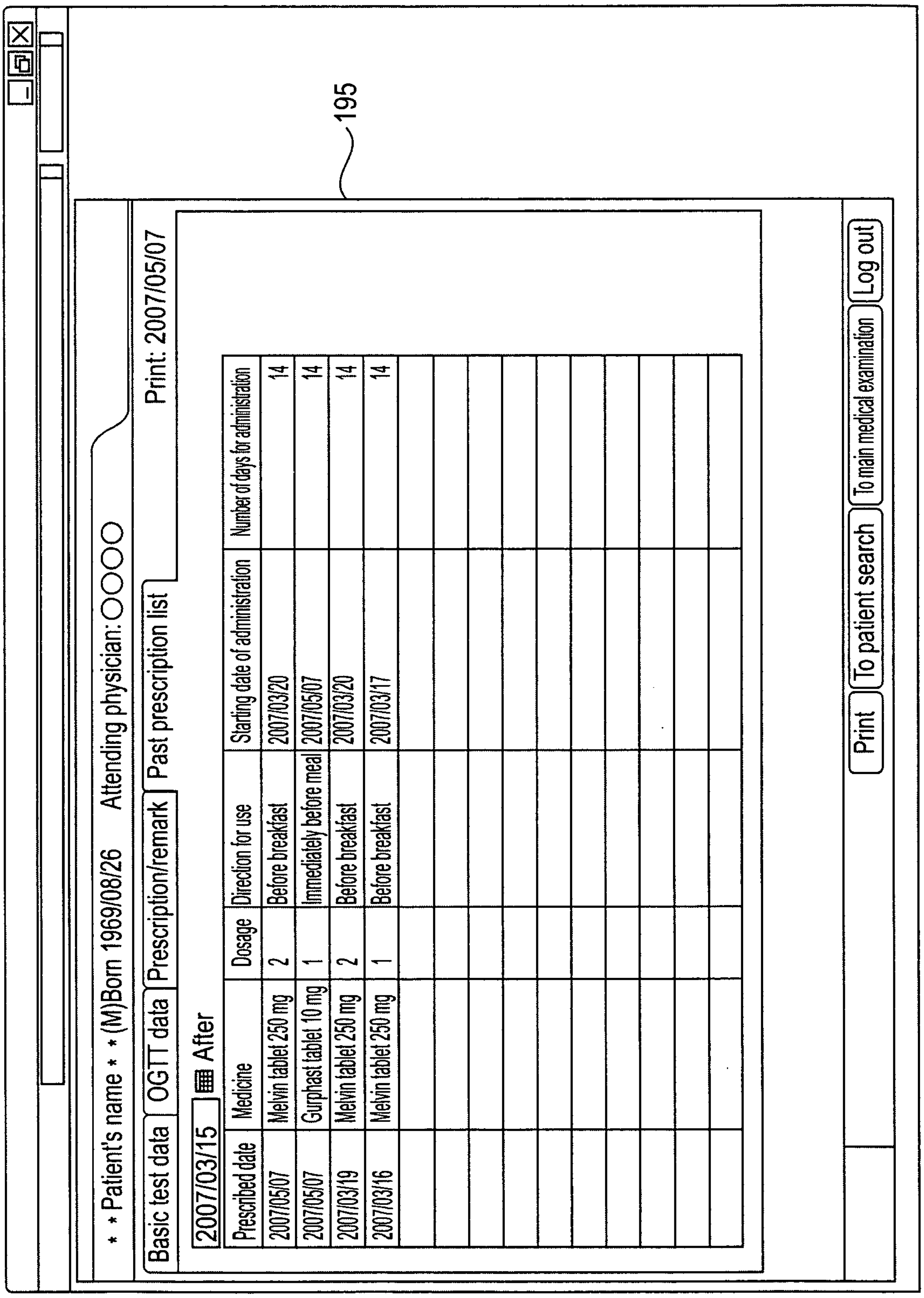
195
* * Patient's name * * (M)Born 1969/08/26 Attending physician: ○○○○
Basic test data
OGTT data
Prescription/remark
Past prescription list
Print: 2007/05/07
2007/03/15
After
Prescribed date
Medicine
Dosage
Direction for use
Starting date of administration
Number of days for administration
2007/05/07
Melvin tablet 250 mg
2
Before breakfast
2007/03/20
14
2007/05/07
Gurphast tablet 10 mg
1
Immediately before meal
2007/05/07
14
2007/03/19
Melvin tablet 250 mg
2
Before breakfast
2007/03/20
14
2007/03/16
Melvin tablet 250 mg
1
Before breakfast
2007/03/17
14
Print
To patient search
To main medical examination
Log out

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 10332704 A
- JP 11296598 A